# EUROPEAN PATENT APPLICATION

(11) **EP 3 835 789 A1**
(43) Date of publication of application: **16.06.2021**
(21) Application number: 19216187.5
(22) Date of filing: 13.12.2019
(51) Int. Cl.: G01N 33/574

(54) **BIOMARKER PANEL FOR DIAGNOSING COLORECTAL CANCER**

(71) Applicant: Deutsches Krebsforschungszentrum, Stiftung des öffentlichen Rechts, 69120 Heidelberg (DE)
(72) Inventor: Brenner, Hermann, 69120 Heidelberg (DE); Bhardwaj, Megha, 69120 Heidelberg (DE); Schrotz-King, Petra, 69120 Heidelberg (DE)
(74) Representative: Kuttenkeuler, David

(57) **Abstract**

The present invention pertains to a method for the diagnosis, prognosis, stratification and/or monitoring of a therapy, of a cancer disease in a patient. The method is based on the determination of the level of one or more biomarkers (a panel) selected from Alpha 1 antitrypsin (AlAT), Apolipoprotein A I (APOAl), Haptoglobin (HP), Leucine rich alpha 2 glycoprotein (LRGi), Serum paraoxonase lactonase 3 (PON3), Apolipoprotein C I (APOCi), Gelsolin (GSN), Hepatocyte growth factor like protein (MSTi), and Transferrin receptor protein 1 (TFRC). The new biomarker panel of the invention allows for diagnosing various cancer diseases and stratifying cancer patients into groups suffering from different stages of the disease, for example various stages of colorectal cancer (CRC). The biomarker panel of the invention further allows for deciding on treatment options of cancer patients as well as for the prediction of therapy outcomes. Furthermore provided are diagnostic kits for performing the methods of the invention.

## Description

### FIELD OF THE INVENTION

The present invention pertains to a method for the diagnosis, prognosis, stratification and/or monitoring of a therapy, of a cancer disease in a patient. The method is based on the determination of the level of one or more biomarkers (a panel) selected from Alpha 1 antitrypsin (A1AT), Apolipoprotein A I (APOA1), Haptoglobin (HP), Leucine rich alpha 2 glycoprotein (LRGi), Serum paraoxonase lactonase 3 (PON₃), Apolipoprotein C I (APOCi), Gelsolin (GSN), Hepatocyte growth factor like protein (MSTi), and Transferrin receptor protein 1 (TFRC). The new biomarker panel of the invention allows for diagnosing various cancer diseases and stratifying cancer patients into groups suffering from different stages of the disease, for example various stages of colorectal cancer (CRC). The biomarker panel of the invention further allows for deciding on treatment options of cancer patients as well as for the prediction of therapy outcomes. Furthermore provided are diagnostic kits for performing the methods of the invention.

### DESCRIPTION

A major step in many aspects of research related to diseases such as cancer is the identification of specific and sensitive biomarkers suitable for the development of effective and improved diagnostic, prognostic and therapeutic modalities. Whilst mass spectrometry, shot gun proteomics, DNA/RNA microarray analyses, and deep sequencing have resulted in an increasing list of reported potential tumor biomarkers, very few have found their way into the clinical validation phase and even fewer are used as reliable therapeutic targets or diagnostic markers.

With 1.85 million incident cases and almost 900,000 deaths per year, colorectal cancer (CRC) is the third most common cancer and second leading cause of cancer mortality globally. Due to the slow progression from precancerous lesions to CRC, early detection could strongly reduce the burden of this disease. Screening programs could help in effectively reducing the incidence and mortality of CRC. However, the participation rates in endoscopy based screening programs are often low due to inconvenience and invasiveness. Moreover, stool test based screening programs suffer from reservation against collection, handling and storage of stool.

Sigmoidoscopy and colonoscopy, the current gold standards for detection of CRC in the distal and total colorectum, respectively, are limited by several disadvantages, such as high costs, limited resources and low compliance. Established non-invasive screening tests are based on stool testing, such as guaiac based faecal occult blood tests (gFOBTs) and faecal immunochemical tests (FITs). However, gFOBTs are limited by low sensitivity and both gFOBTs and FITs face limitations in adherence related to the need of stool collection.

The human proteome consists of approximately 20,000 proteins and has been explored for diagnostic biomarkers by a rapidly increasing number of studies. The detection of low abundant putative cancer biomarkers in blood is often impaired by the high complexity and wide dynamic concentration range of proteins in blood. However, with the advancements in the field of targeted proteomics, the quantitation and verification of these low abundant markers is possible even with lower sample volumes.

The protein biomarkers of the current invention were quantified using Liquid chromatography/multiple reaction monitoring-mass spectrometry (LC-MRM/MS), a target specific and technically sensitive high throughput measure that enables simultaneous quantitation of various proteins. LC-MRM/MS can effectively quantitate even low abundant markers, as it possesses high multiplexing capacity, high analytic specificity of up to five dimensions and high analytical sensitivity in the nanogram/ml range.

The quantitation of selected targets in LC-MRM/MS is facilitated by pre-defined pairs of precursor and product ions. The peptides selected for LC-MRM/MS uniquely identify each target protein and the measurement of mass and fragmentation spectra of peptides is highly accurate because of sequence specific digestion of proteins. Furthermore, the use of a triple-quadrupole facilitates high technical specificity by allowing only a selected peptide and later only a specific fragment to pass through the first and second quadrupoles, respectively.

Most previous studies on blood-based biomarkers for CRC suffer from a lack of validation of signatures in true screening settings.

WO2012006681 (A1) provides a method for diagnosing colorectal cancer by determining the presence and/or level of biomarkers selected from IL-8, IGFBP2, MAC2BP, M2PK, IL-13, DKK-3, EpCam, MIP1β, TGFβ1, and TIMP-1. The invention also relates to diagnostic kits comprising reagents for determining the presence and/or level of the biomarkers and methods of detecting or diagnosing colorectal cancer, but no validation of the biomarker panel in a true screening setting had been performed.

WO2015047910 (A1) provides means for a biomarker for detection of early stages (I and II) of colorectal cancer and methods for in vitro diagnosis of early stages (I and II) of colorectal cancer.

A study perfomed by Surinova, et al. (2015, Prediction of colorectal cancer diagnosis based oncirculating plasma proteins. Embo Molecular Medicine, 2015. 7(9): p. 1166-1178) used Selected/Multiple RM-MS based approaches to identify six maker algorithms with an AUC of 0.84. Furthermore, the study by Jones, et al. (2016. A Plasma-Based Protein Marker Panel for Colorectal Cancer Detection Identified by Multiplex Targeted Mass Spectrometry. Clinical Colorectal Cancer, 2016. 15(2): p. 186) used Selected/Multiple RM-MS based approaches to identify a thirteen maker algorithm with an AUC of 0.91. However, unlike the current invention, the external validation set of these aforementioned studies consisted of CRC cases that were recruited in clinical settings and the performance of these signatures in participants from screening colonoscopy remains unknown.

Blood-based screening markers for CRC that have only been evaluated in samples from clinical settings, i.e. in participants that were recruited directly from hospitals, are problematic because the CRC cases typically include a higher proportion of cases in advanced tumor stages. Furthermore, participants recruited in clinical settings may have undertaken some diagnostic or early therapeutic procedures, which may influence potential biomarkers and might lead to overestimation of differences from biomarker levels in healthy controls and hence of diagnostic performance. Additionally, confounding may result from non-comparability of cases and controls with respect to other factors, such as other medical conditions, setting of recruitment, or pre-analytical handling of blood samples.

Unlike the previous studies, validation of protein signatures of this invention was exclusively done among prospectively recruited participants of screening colonoscopy who represent the target population for population-based screening. Conduction of screening colonoscopy in all participants furthermore enabled identification of participants with advanced adenoma, which is another important target of CRC screening. Although participants in the discovery and validation sets used for the current invention were selected from three different studies, the collection, handling, processing and storage of the samples across the three studies were performed with similar standardized operating procedure.

It is critical to identify biomarkers and to evaluate their diagnostic performance in a true screening setting. Hence, the algorithms of this invention were first identified in a discovery set. Subsequently, the performance of the markers was validated in an independent validation set from a cohort of 9245 participants of screening colonoscopy, i.e. participants that were prospectively recruited in a true screening setting. Importantly, the use of an independent external validation set exclusively including participants of screening colonoscopy resulted in a better diagnostic performance for detecting advanced adenoma than was previously reported for the only FDA approved blood-based test for CRC detection.

The study of this invention was performed on a highly target specific LC-MRM/MS method in a two stage design, with validation performed exclusively in a true screening setting. Additionally, advanced statistical machine learning algorithms were applied in form of LASSO along with .632+ bootstrap to control for overoptimism simultaneously on 270 protein biomarkers to evaluate all combinations. Moreover, signatures for stage specific detection of CRC were derived apart from an algorithm for overall prediction of CRC presence.

Due to the continuing need for quick, but sensitive and specific cancer diagnostics, it is an object of the present invention to provide a novel approach for a simple and minimal invasive but specific and sensitive test system for the diagnosis and/or monitoring of various cancer diseases. Blood-based protein biomarker signatures might be used as an alternative or supplement for detection of colorectal cancer (CRC) for population-based screening. Thus, the problem to be solved by this invention is to derive a protein biomarker signature for detection of CRC and its precursors advanced adenomas (AA). It is an objection of this invention to provide a non-invasive, easy applicable and cheap blood-based test for diagnosis and/or monitoring of various stages of CRC. Yet another problem to be solved by this invention is to identify, evaluate and validate novel protein biomarkers and biomarker panels for stage specific detection of CRC, and/or for prognosis of CRC, and/or for use in the development of novel therapeutics. Due to their non-invasive nature and ease of application in routine medical practice, blood-based tests could ensure high levels of adherence when applied as primary screening tools in population-based CRC screening, especially for individuals who are reluctant to stool sampling.

### BRIEF DESCRIPTION OF THE INVENTION

Generally, and by way of brief description, the main aspects of the present invention can be described as follows:

In **a first aspect,** the invention pertains to a method for the diagnosis, prognosis, stratification and/or monitoring of a therapy, of a cancer disease in a subject, comprising the steps of:
**(a)** Providing a biological sample from the subject,
**(b)** Determining the level of at least one or more biomarker selected from the group consisting of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON₃), Apolipoprotein C I (APOCi), Gelsolin (GSN), Hepatocyte growth factor like protein (MSTi), and Transferrin receptor protein 1 (TFRC), in the biological sample,
wherein a differential level of the at least one or more biomarker in the biological sample from the subject as determined in step (b) compared to a healthy control or reference value is indicative for the presence of a cancer disease in the subject.

In a **second aspect,** the invention pertains to a method **of selecting a therapy for treating, and/or for treating, colorectal cancer in a subject,** said method comprising the steps of:
**(a)** Obtaining a biological sample from the subject,
**(b)** Determining the level of at least one or more biomarker selected from the group consisting of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON₃), Apolipoprotein C I (APOCi), Gelsolin (GSN), Hepatocyte growth factor like protein (MST1), and Transferrin receptor protein 1 (TFRC), in the biological sample, and
**(c)** Deciding in favor or against a therapy,
**(d)** wherein, if (i) no differential level of the at least one or more biomarker in the biological sample from the subject as determined in step **(b)** compared to a healthy control or reference value is determined, it is decided against said therapy in step **(c),** and wherein if **(ii)** a differential level of the at least one or more biomarker in the biological sample from the subject as determined in step **(b)** compared to a healthy control or reference value is determined to be indicative for early stage o/I/II colorectal cancer, it is decided in favor of a surgical type of therapy in step **(c),** thereby treating the colorectal cancer in the subject, and if **(iii)** a differential level of the at least one or more biomarker in the biological sample from the subject as determined in step **(b)** compared to a healthy control or reference value is determined to be indicative for late stage III/IV colorectal cancer, it is decided in favor of a surgical type of therapy, and/or a non-surgical type of therapy comprising a therapeutic agent in step **(c),** thereby treating the colorectal cancer in the subject.

In **a third aspect,** the invention pertains to a method for determining a blood protein marker status in a patient preferably a cancer patient, more preferably a colorectal cancer patient, comprising the steps of:
**(a)** Providing a biological sample from the subject,
**(b)** Determining the level of at least one or more biomarker selected from the group consisting of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON₃), Apolipoprotein C I (APOCi), Gelsolin (GSN), Hepatocyte growth factor like protein (MSTi), and Transferrin receptor protein 1 (TFRC), in the biological sample, and
**(c)** Determining the blood protein marker status from the determined levels in step **(b).**

In **a fourth aspect,** the invention pertains to a method for evaluating the treatment success of a patient who received a cancer treatment comprising
**(a)** Providing a biological sample from said patient who received a cancer treatment,
**(b)** Determining the level of at least one or more biomarker selected from the group consisting of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON₃), Apolipoprotein C I (APOC1), Gelsolin (GSN), Hepatocyte growth factor like protein (MST1), and Transferrin receptor protein 1 (TFRC), in the biological sample, and
**(c)** Comparing the level of said at least one biomarker as determined in (b) with a reference,
wherein a decrease or an increase of the level of at least one or more biomarker in the biological sample from said subject compared to said reference sample or reference value is an indication for the patient's response to said treatment.

In **a fifth aspect,** the invention pertains to a method of treating cancer in a patient, comprising a method according to the previous aspects, and treating and/or preventing said cancer or the recurrence of said cancer in said patient by administering to the subject a therapeutically effective amount of a therapeutic agent.

In **a sixth aspect,** the invention pertains to a diagnostic kit for performing the methods of the invention.

In **a seventh aspect,** the invention pertains to a use of an antibody, antigen binding derivative, or antigenic fragment thereof, directed to any one of the protein biomarkers selected from A1AT, APOA1, HP, LRG1, PON₃, APOC1, GSN, MST1, and TFRC, in the performance of a method according to the previous aspects.

In **a eighth aspect,** the invention pertains to a use of a biomarker selected from the group consisting of A1AT, APOA1, HP, LRG1, PON₃, APOC1, GSN, MST1, and TFRC, in a screening method for identifying cancer therapeutic.

### DETAILED DESCRIPTION OF THE INVENTION

In the following, the elements of the invention will be described. These elements are listed with specific embodiments, however, it should be understood that they may be combined in any manner and in any number to create additional embodiments. The variously described examples and preferred embodiments should not be construed to limit the present invention to only the explicitly described embodiments. This description should be understood to support and encompass embodiments which combine two or more of the explicitly described embodiments or which combine the one or more of the explicitly described embodiments with any number of the disclosed and/or preferred elements. Furthermore, any permutations and combinations of all described elements in this application should be considered disclosed by the description of the present application unless the context indicates otherwise.

The above problem is solved in a first aspect by a method for the diagnosis, prognosis, stratification and/or monitoring of a therapy, of a cancer disease in a subject, comprising the steps of:
**(a)** Providing a biological sample from the subject,
**(b)** Determining the level of at least one or more biomarker selected from the group consisting of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON₃), Apolipoprotein C I (APOCi), Gelsolin (GSN), Hepatocyte growth factor like protein (MSTi), and Transferrin receptor protein 1 (TFRC), in the biological sample,
wherein a differential level of the at least one or more biomarker in the biological sample from the subject as determined in step (b) compared to a healthy control or reference value is indicative for the presence of a cancer disease in the subject.

The human protein Alpha-i-antitrypsin (A1AT) is encoded by the gene *SERPINA1.* The protein can be found in the UniProt database under the accession number P01009 (AiAT_HUMAN). Preferably, such protein is known to have an isoform amino acid sequence shown in SEQ ID NO: 1.

The human protein Apolipoprotein A-I (APOA1) is encoded by the gene *APOA1.* The protein can be found in the UniProt database under the accession number P02647 (APOA1_HUMAN). Preferably, such protein is known to have an isoform amino acid sequence shown in SEQ ID NO: 2.

The human protein Haptoglobin (HP) is encoded by the gene *HP.* The protein can be found in the UniProt database under the accession number P00738 (HPT_HUMAN). Preferably, such protein is known to have an isoform amino acid sequence shown in SEQ ID NO: 3.

The human protein Leucine rich alpha 2 glycoprotein (LRG1) is encoded by the gene *LRG1.* The protein can be found in the UniProt database under the accession number P02647 P02750 (A2GL_HUMAN). Preferably, such protein is known to have an isoform amino acid sequence shown in SEQ ID NO: 4.

The human protein Serum paraoxonase lactonase 3 (PON₃) is encoded by the gene *PON₃.* The protein can be found in the UniProt database under the accession number Q15166 (PON_{3_}HUMAN). Preferably, such protein is known to have an isoform amino acid sequence shown in SEQ ID NO: 5.

The human protein Apolipoprotein C I (APOC1) is encoded by the gene *APOC1.* The protein can be found in the UniProt database under the accession number P02654 (APOC1_HUMAN). Preferably, such protein is known to have an isoform amino acid sequence shown in SEQ ID NO: 6.

The human protein Gelsolin (GSN) is encoded by the gene *GSN.* The protein can be found in the UniProt database under the accession number P06396 (GELS_HUMAN). Preferably, such protein is known to have an isoform amino acid sequence shown in SEQ ID NO: 7.

The human protein Hepatocyte growth factor like protein (MST1) is encoded by the gene *MST1.* The protein can be found in the UniProt database under the accession number P26927 (HGFL_HUMAN). Preferably, such protein is known to have an isoform amino acid sequence shown in SEQ ID NO: 8.

The human protein Transferrin receptor protein 1 (TFRC) is encoded by the gene *Transferrin receptor protein 1 (TFRC).* The protein can be found in the UniProt database under the accession number P02786 (TFRi_HUMAN). Preferably, such protein is known to have an isoform amino acid sequence shown in SEQ ID NO: 9.

The terms "diagnosis" or "diagnostic", or similar expressions, in context of the present invention shall refer to identifying the presence or absence, or identifying the nature of a pathologic condition in a subject. Diagnostic methods differ in their sensitivity and specificity. The "sensitivity" of a diagnostic assay is a measure of the percentage of diseased individuals who test positive (percent of "true positives"). Diseased individuals not detected by the assay are "false negatives." Subjects, who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

The term "prognosis" refers to a forecast as to the probable outcome of a disease as well as the prospect of recovery from a disease as indicated by the nature and symptoms of the case. Accordingly, a negative or poor prognosis is defined by a lower post-treatment survival term or survival rate. Conversely, a positive or good prognosis is defined by an elevated post-treatment survival term or survival rate. Usually prognosis is provided as the time of progression free survival or overall survival.

The term "stratification" or "stratifying" for the purposes of this invention refers to the division of a patient population into patient subpopulations on the basis of specified criteria. More particularly, it refers to the division of a cohort of subjects or patients into at least two groups on the basis of specific criteria, which in the context of the present invention comprise or consist of the determined levels of the biomarker of the panel of the present invention. The method according to the invention renders possible decisions for the treatment and therapy of the patient, whether it is the hospitalization of the patient, the use, effect and/or dosage of one or more drugs, a therapeutic measure or the monitoring of a course of the disease and the course of therapy or etiology or classification of a disease, e.g., into a new or existing subtype or the differentiation of diseases and the patients thereof. Particularly with regard to colorectal cancer, "stratification" means in this context a classification of a colorectal cancer as early or late stage colorectal cancer.

The term "monitoring a therapy" for the purpose of the present invention means to observe disease progression in a subject who receives a cancer therapy. In other words, the subject during the therapy is regularly monitored for the effect of the applied therapy, which allows the medical practitioner to estimate at an early stage during the therapy whether the prescribed treatment is effective or not, and, therefore, to continue, adjust or end the treatment regime accordingly.

As used herein, the term "subject" or "patient" refers to any animal (e.g., a mammal), including, but not limited to, humans, non-human primates, rodents, and the like, which is to be the recipient of a particular treatment or diagnostic method. Typically, the terms "subject" and "patient" are used interchangeably herein in reference to a human subject. As used herein, the term "subject suspected of having cancer" refers to a subject that presents one or more symptoms indicative of a cancer (e.g., a noticeable lump or mass). A subject suspected of having cancer may also have one or more risk factors. A subject suspected of having cancer has generally not been tested for cancer. However, a "subject suspected of having cancer" encompasses an individual who has received an initial diagnosis (e.g., a CT scan showing a mass, or in case of colorectal cancer a positive observation during colonoscopy) but for whom the sub-type or stage of cancer is not known. The term further includes people who once had cancer (e.g., an individual in remission), and people who have cancer and are suspected to have a metastatic spread of the primary tumor. In this regard the present invention is also applicable as follow-up care for monitoring a subject for a reoccurrence of the cancer.

In a particularly preferred embodiment, the subject is a mammal, such as a mouse, a rat, a guinea pig, a rabbit, a cat, a dog, a monkey, or a human, preferably a human, such as a human patient, more preferably a human patient suffering from a disease, such as cancer, and in need of a therapy, most preferably a human patient suffering from colorectal cancer and in need of a therapy.

The terms "cancer" and "cancer cells" refer to any cells that exhibit uncontrolled growth in a tissue or organ of a multicellular organism. Particularly preferred cancers in context of the present invention are selected from colorectal cancer, advanced adenoma, pancreatic cancer, gastric cancer, breast cancer, lung cancer, prostate cancer, hepatocellular cancer, cervical cancer, ovarian cancer, liver cancer, bladder cancer, cancer of the urinary tract, thyroid cancer, renal cancer, carcinoma, melanoma, leukemia, and brain cancer. More preferably, the cancer is colorectal cancer, and/or advanced adenoma, and wherein said colorectal cancer is early stage o/I/II colorectal cancer, or late stage III/IV colorectal cancer.

As used herein, the term "colorectal cancer" includes the well-accepted medical definition that defines colorectal cancer as a medical condition characterized by cancer of cells of the intestinal tract below the small intestine (i.e., the large intestine (colon), including the cecum, ascending colon, transverse colon, descending colon, sigmoid colon, and rectum). Additionally, as used herein, the term "colorectal cancer" also further includes medical conditions, which are characterized by cancer of cells of the duodenum and small intestine (jejunum and ileum).

As used herein, the terms "gastric cancer" or "stomach cancer" refer to cancers of the stomach. The most common types of gastric cancer are carcinomas, such as but not limited to, adenocarcinomas, affecting the epithelial cells of the stomach. Stomach cancers may additionally include, for example, sarcomas affecting the connective tissue of the stomach and blastomas affecting the blast tissue of the stomach.

The term "pancreatic cancer" encompasses benign or malignant forms of pancreatic cancer, as well as any particular type of cancer arising from cells of the pancreas (e.g., duct cell carcinoma, acinar cell carcinoma, papillary carcinoma, adenosquamous carcinoma, undifferentiated carcinoma, mucinous carcinoma, giant cell carcinoma, mixed type pancreatic cancer, small cell carcinoma, cystadenocarcinoma, unclassified pancreatic cancers, pancreatoblastoma, and papillary-cystic neoplasm, and the like.).

The term "biological sample" as used herein refers to a sample that was obtained and may be assayed for any one of the biomarkers as disclosed with the present invention, or their gene expression. The biological sample can include a biological fluid (e.g., blood, cerebrospinal fluid, urine, plasma, serum), tissue biopsy, and the like. In some embodiments, the sample is a tissue sample, for example, a tumor tissue sample, and may be fresh, frozen, or archival paraffin embedded tissue. Preferred samples for the purposes of the present invention are bodily fluids, in particular blood or plasma samples.

In a particularly preferred embodiment, the biological sample is a sample of a subject comprising a tissue sample or a body liquid sample, such as a blood sample, a serum sample, a plasma sample, a sample of a group of cells from a tumor, a tumor tissue, a urine sample, a lymph fluid sample, a pleural fluid sample, or a brain liquor sample, preferably wherein the biological sample is a blood sample, such as a plasma sample.

A "biomarker" or "marker" in the context of the present invention refers to an organic biomolecule, particularly a polypeptide, which is differentially present in a sample taken from subjects having a certain condition as compared to a comparable sample taken from subjects who do not have said condition (e.g., negative diagnosis, normal or healthy subject, or non-cancer patients, depending on whether the patient is tested for cancer, or metastatic cancer). For example, a marker can be a polypeptide or polysaccharide (having a particular apparent molecular weight) which is present at an elevated or decreased level in samples of cancer patients compared to samples of patients with a negative diagnosis.

The term "determining the level of' a biomarker in a sample, control or reference, as described herein shall refer to the quantification of the presence of said biomarker in the tested sample. For example, the concentration of the biomarker in said sample may be directly quantified via measuring the amount of protein/polypeptide/polysaccharide as present in the tested sample. However, also possible is to quantify the amount of biomarker indirectly via assessing the gene expression of the encoding gene of the biomarker, for example by quantification of the expressed mRNA encoding for the respective biomarker protein. The present invention shall not be restricted to any particular method for determining the level of a given biomarker, but shall encompass all means that allow for a quantification, and/or estimation, of the level of said biomarker, either directly or indirectly. "Level" in the context of the present invention is therefore a parameter describing the absolute amount of a biomarker in a given sample, for example as absolute weight, volume, or molar amounts; or alternatively "level" pertains to the relative amounts, and preferably to the concentration of said biomarker in the tested sample, for example mol/l, g/l, g/mol etc. In preferred embodiments the "level" refers to the concentration of the tested biomarkers in g/l.

In the context of this invention, a reference value shall correspond to a sample obtained from a healthy individual or obtained from a tumor-adjacent tissue from a subject suffering from a tumor disease. In certain embodiments the reference value may also be derived from a sample obtained the same subject at an earlier time point.

"Increase" of the level of a biomarker in a sample compared to a control shall in preferred embodiments refer to a statistically significant increase in preferred aspects of the invention.

In alternative embodiments of the invention, certain biomarkers as disclosed herein may also be significantly decreased in the event of a cancer disease in a subject.

In a preferred embodiment the method of the herein disclosed invention is a non-invasive, minimally invasive, *ex-vivo* or *in-vitro* method. If the herein described diagnostic methods are non-invasive, the term "providing a biological" sample shall preferably not be interpreted to include a surgical procedure conducted at the subject.

To date, no single blood biomarker qualifying for mass screening has been identified. The combination of multiple markers might be a more promising approach to achieve the necessary sensitivity and specificity for application in mass screening. Although other marker panels were tested in the prior art, the apparent differences to the panel as provided herein can be explained by the fact that those prior art studies were done in a clinical setting and did not apply any adjustment for over-optimism (not doing so would have yielded an even higher AUC in the inventor's study). The above mentioned limitations were also shared by many other studies regarding blood biomarkers for CRC detection. For reasons outlined in detail in the introduction, it is a critical issue to identify and evaluate biomarkers in samples from screening settings in order to obtain valid performance characteristics under screening conditions. Furthermore, as demonstrated herein, correction for overfitting (cross-validation, bootstrap techniques) and/or external validation are also indispensable to adjust for potential overestimation of diagnostic performance. Hence, the marker panel of the present invention is advantageous over previous prior art panels.

The biomarker panels as disclosed herein are particularly useful in a cancer screening setting. Cancer screening in the herein disclosed invention shall refer to a procedure where a subject for which no diagnosis was established is tested for the presence of the cancer disease. This shall not be interpreted to exclude the use of the biomarker of the invention for a diagnosis of a subject who was already diagnosed to suffer from a cancer disease. Non limiting examples for such an application are confirmation of a diagnosis, monitoring or treatment success or monitoring reoccurrence of a cancer in a subject that already received a treatment and wherein cancer is in remission or was cured.

In one preferred embodiment, the biomarker is a protein biomarker, and the method is characterized in that the determining the level of the at least one or more biomarker involves determining the level of a protein of the at least one or more biomarker in the biological sample.

A further preferred embodiment relates to the method of this invention, wherein the method is a screening method for establishing a first diagnosis of cancer in the subject.

Preferred embodiments of the present invention pertain to panels of a plurality of biomarkers as identified herein for the diagnostic purposes as described. The advantage of combing the biomarkers disclosed herein is an increased sensitivity and/or specificity of the disclosed assays. Hence a preferred embodiment of the invention pertains to the herein disclosed method wherein step (b) comprises determining the level of at least two, three, four, five, six, seven, eight or nine biomarkers in the biological sample. More preferred is that at least three biomarkers are used. More preferred is that at least four biomarkers are used. More preferred is that at least five biomarkers are used. More preferred is that at least six biomarkers are used. More preferred is that at least seven biomarkers are used. More preferred is that at least eight biomarkers are used. More preferred is that at least nine biomarkers are used.

In course of the present invention, 270 protein markers were measured by liquid chromatography-multiple reaction monitoring/mass spectrometry (LC-MRM/MS) in plasma samples of discovery and validation sets in a two-stage design. In the discovery set consisting of 100 newly diagnosed CRC cases and 100 age and sex matched controls free of neoplasm at screening colonoscopy, the algorithms predicting the presence of early or late stage CRC were derived by Lasso regression and .632+ bootstrap. The prediction algorithms were then externally validated in an independent validation set consisting of participants of screening colonoscopy including 56 participants with CRC, 99 with advanced adenoma (AA) and 99 controls without any colorectal neoplasms. The peptide concentration for all proteins measured was reported in fmol/µl of plasma in the samples and the coefficient of variance was <20% for all samples. 95 proteins had zero values (i.e. the abundance of NAT peptides was below limit of quantitation) in all samples in the discovery set and in all samples in the validation set. Consequently, the analyses focused on 175 proteins detected in both the discovery and the validation set.

In one preferred embodiment of the herein disclosed invention the level of at least one of A1AT, APOA1, HP, LRG1, PON₃, APOC1, GSN, MST1, or TFRC, in the biological sample, is determined.

In another preferred embodiment of the herein disclosed invention the level of at least two of A1AT, APOA1, HP, LRG1, PON₃, APOC1, GSN, MST1, or TFRC, in the biological sample, is determined. Such combinations may include A1AT and one or more of APOA1, HP, LRG1, PON₃, APOC1, GSN, MST1, or TFRC; or may include APOA1, and one or more of A1AT, HP, LRG1, PON₃, APOC1, GSN, MST1, or TFRC; or may include HP, and one or more of A1AT, APOA1, LRG1, PON₃, APOC1, GSN, MST1, or TFRC; or may include LRG1, and one or more of A1AT, APOA1, HP, PON₃, APOC1, GSN, MST1, or TFRC; or may include PON₃, and one or more of A1AT, APOA1, HP, LRG1, APOC1, GSN, MST1, or TFRC; or may include APOC1, and one or more of A1AT, APOA1, HP, LRG1, PON₃, GSN, MST1, or TFRC; or may include GSN, and one or more of A1AT, APOA1, HP, LRG1, PON₃, APOC1, MST1, or TFRC; or may include MST1, and one or more of A1AT, APOA1, HP, LRG1, PON₃, APOC1, GSN, or TFRC; or may include TFRC, and one or more of A1AT, APOA1, HP, LRG1, PON₃, APOC1, GSN, or MST1.One specifically preferred panel for use in context of the herein disclosed invention comprises the selection of at least 3, 4, 5, 6, 7, 8, or 9 biomarkers selected from the group of least A1AT, APOA1, HP, LRG1, PON₃, APOC1, GSN, MST1, and TFRC in the biological sample. Most preferred in this aspect is that at least the biomarkers HP, LRG1, and PON₃, and optionally any of the remaining biomarkers is included in this panel.

In context of the herein disclosed invention a differential level of a biomarker selected from A1AT, APOA1, HP, LRG1, PON₃, APOC1, GSN, MST1, and TFRC, can be a higher level of that biomarker in a positive diagnosis. On the other hand, a differential level of a biomarker selected from A1AT, APOA1, HP, LRG1, PON₃, APOC1, GSN, MST1, and TFRC, can also be a lower level in a positive diagnosis.

The skilled artisan will understand that numerous methods may be used to select a threshold or reference value for a particular marker or a plurality of markers. In diagnostic aspects, a threshold value may be obtained by performing the assay method on samples obtained from a population of patients having a certain type of cancer, and from a second population of subjects who do not have cancer. For prognostic or treatment monitoring applications, a population of patients, all of which have, for example, CRC, may be followed for the time period of interest (e.g., six months following diagnosis or treatment, respectively), and then dividing the population into two groups: a first group of subjects that progress to an endpoint (e.g., recurrence of disease, death); and a second group of subjects that did not progress to the end point. These are used to establish "low risk" and "high risk" population values for the marker(s) measured, respectively. Other suitable endpoints include, but are not limited to, 5-year mortality rates or progression to metastatic disease.

Once these groups are established, one or more thresholds may be selected that provide an acceptable ability to predict diagnosis, prognostic risk, treatment success, etc. In practice, Receiver Operating Characteristic curves, or "ROC" curves, are typically calculated by plotting the value of a variable versus its relative frequency in two populations (called arbitrarily "disease" and "normal" or "low risk" and "high risk" for example). For any particular marker, a distribution of marker levels for subjects with and without a disease may overlap. Under such conditions, a test does not absolutely distinguish "disease" and "normal" with 100% accuracy, and the area of overlap indicates where the test cannot distinguish "disease" and "normal." A threshold is selected, above which (or below which, depending on how a marker changes with the disease) the test is considered to be "positive" and below which the test is considered to be "negative." The area under the ROC curve (AUC) is a measure of the probability that the perceived measurement may allow correct identification of a condition.

Additionally, thresholds may be established by obtaining an earlier marker result from the same patient, to which later results may be compared. In some aspects, the individuals act as their own "control group." In markers that increase with disease severity or prognostic risk, an increase over time in the same patient can indicate a worsening of disease or a failure of a treatment regimen, while a decrease over time can indicate remission of disease or success of a treatment regimen.

In some embodiments, multiple thresholds or reference values may be determined. This can be the case in so-called "tertile," "quartile," or "quintile" analyses. In these methods, the "disease" and "normal" groups (or "low risk" and "high risk") groups can be considered together as a single population, and are divided into 3, 4, or 5 (or more) "bins" having equal numbers of individuals. The boundary between two of these "bins" may be considered "thresholds." A risk (of a particular diagnosis or prognosis for example) can be assigned based on which "bin" a test subject falls into.

In a preferred embodiment of the herein disclosed invention a differential level of a biomarker panel comprising at least HP, LRG1, and PON₃ is indicative for early stage o/I/II colorectal cancer. Hence, a particularly preferred embodiment of the present invention relates to the determining a differential level of at least HP, LRG1, and PON₃, in the biological sample, wherein the diagnosed cancer disease is colorectal cancer, and/or advanced adenoma, more preferably colorectal cancer and wherein the colorectal cancer is early stage o/I/II colorectal cancer.

A further preferred embodiment of the present invention relates to the determining of a differential level of at least A1AT, APOA1, HP, LRG1, and PON₃ in the biological sample, preferably wherein the cancer disease is colorectal cancer.

Yet another preferred embodiment of the present invention relates to the determining of a differential level of at least A1AT, APOA1, HP, LRG1, APOC1, GSN, MST1, and TFRC in the biological sample, wherein the cancer disease is colorectal cancer, and more preferably wherein the colorectal cancer is late stage III/IV colorectal cancer.

It is preferred that the analysis of the marker panel in step (b) of the diagnostic method of the invention is characterized in that the tested marker panel has an apparent area under the curve (AUC) at 95% confidence interval (CI) of at least 60%, preferably at least 65% or more preferably at least 70%. How to determine the AUC is known to the skilled artisan. Alternatively or additionally, the panel of the invention may be characterized by a sensitivity of at least 75%, preferably at least 80%, and a specificity of at least 40%, preferably at least 50%, more preferably at least 60%.

All numeric values are herein assumed to be modified by the term "about," whether or not explicitly indicated. The term "about" generally refers to a range of numbers that one of skill in the art would consider equivalent to the recited value (i.e., having the same function or result). In many instances, the terms "about" may include numbers that are rounded to the nearest significant figure. In particularly preferred embodiments of the invention the term "about" may refer to a deviation of the respective numeric value of a maximum of 20% of the numerical value, however more preferred is 15%, 10%, 5% and most preferred is 4%, 3%, 2%, and most preferred is 1%.

In all aspects and embodiments of the present invention it may be preferred that the level of said at least one biomarker in said sample is determined by means of a nucleic acid detection method or a protein detection method. However, nucleic acid detection methods are only applicable where an expressed protein is the biomarker. Generally all means shall be comprised by the present invention which allow for a quantification of the expression of any one of the herein disclosed biomarker. Therefore also promoter analysis and procedures assessing the epigenetic status of a gene locus encoding a protein biomarker of the invention are comprised by the herein described invention.

Detection methods that are preferred in context of the herein described invention are methods, wherein the level of said at least one biomarker in said sample is determined by means of a detection method selected from the group consisting of mass spectrometry, mass spectrometry immunoassay (MSIA), antibody-based protein chips, 2-dimensional gel electrophoresis, stable isotope standard capture with anti-peptide antibodies (SISCAPA), high-performance liquid chromatography (HPLC), western blot, cytometry bead array (CBA), protein immuno-precipitation, radio immunoassay, ligand binding assay, and enzyme-linked immunosorbent assay (ELISA). More preferably, the biomarkers of the present invention are detected using one or more binding agents or antigen binding proteins, such as antibodies, preferably wherein the biomarkers are detected by western blot, ELISA, Proximity Extension Assay, or mass-spectrometrically. Suitable alternative detection methods for quantification of a biomarker of the invention are known to the skilled artisan.

A further aspect of the present invention relates to a method of selecting a therapy for treating, and/or for treating colorectal cancer in a subject, said method comprising the steps of:
**(a)** Obtaining a biological sample from the subject,
**(b)** Determining the level of at least one or more biomarker selected from the group consisting of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON₃), Apolipoprotein C I (APOCi), Gelsolin (GSN), Hepatocyte growth factor like protein (MSTi), and Transferrin receptor protein 1 (TFRC), in the biological sample, and
**(c)** Deciding in favor or against a therapy,
wherein, if (i) no differential level of the at least one or more biomarker in the biological sample from the subject as determined in step (b) compared to a healthy control or reference value is determined, it is decided against said therapy in step (c), and wherein if (ii) a differential level of the at least one or more biomarker in the biological sample from the subject as determined in step (b) compared to a healthy control or reference value is determined to be indicative for early stage o/I/II colorectal cancer, it is decided in favor of a surgical type of therapy in step (c), thereby treating the colorectal cancer in the subject, and if (iii) a differential level of the at least one or more biomarker in the biological sample from the subject as determined in step (b) compared to a healthy control or reference value is determined to be indicative for late stage III/IV colorectal cancer, it is decided in favor of a surgical type of therapy, and/or a non-surgical type of therapy comprising a therapeutic agent in step (c), thereby treating the colorectal cancer in the subject.

In a preferred embodiment of the present invention, it is decided in favor of a surgical type of therapy (according to (ii) above) if step (b) comprises determining a differential level of at least HP, LRG1, and PON₃, in the biological sample. In the context of this invention, the detection of a differential level of at least HP, LRG1, and PON₃, in the biological sample, is indicative for early stages (I and II) of CRC, and a surgical type of therapy is the preferred treatment option for early stages (I and II) of CRC.

In an additionally preferred embodiment of the present invention, it is decided in favor of a surgical type of therapy, and/or a non-surgical type of therapy comprising a therapeutic agent (according to (iii) above) if step (b) comprises determining a differential level of at least A1AT, APOA1, HP, LRG1, APOC1, GSN, MST1, and TFRC, in the biological sample. In the context of this invention, the detection of a differential level of at least A1AT, APOA1, HP, LRG1, APOC1, GSN, MST1, and TFRC, in the biological sample, is indicative for late stages (III and IV) of CRC, and a surgical type of therapy, and/or a non-surgical type of therapy is the preferred treatment option for late stages (III and IV) of CRC.

Based on the determination of biomarkers of this invention, the choice of treatment can vary. In most cases of AA and CRC, a surgical type of resection of the tumor is the first choice. In some cases of early stages CRC (such as stage I and/or II), a surgical resection of the tumor without additional chemoradiation can be sufficient to treat said cancer disease.

In later stages of CRC, surgical resection in combination with adjuvant or neo-adjuvant chemoradiation or chemotherapy shall be the choice of treatment, e.g., in stages III and IV CRC, compared to early stages (I and II) of CRC, where surgery alone is the choice of treatment in most cases. In some rare cases of early stages (I and II) of CRC, in particular in stage II CRC, adjuvant chemotherapy may be used. The reason for this might be that the patient or the family of the patient has a history of cancer diseases, or a predisposition for cancer diseases. Importantly, auxiliary chemotherapy can be effective in preventing the spread of a metastatic disease that comes from a colorectal cancer that has been surgically removed.

In the context of this invention, chemotherapy shall be understood as a cancer treatment with an antineoplastic drug or with a combination of such drugs. The chemotherapy used in the context of this invention shall preferably be suitable for treating advanced adenoma and/or colorectal cancer. In general, chemotherapy normally acts by eliminating rapidly dividing cells, which is one of the main properties of most cancer cells. For the treatment of colorectal cancer patients, chemotherapy preferably comprises the administration of fluoropyrimidine compounds, which are thought to act as antimetabolites. Examples of fluoropyrimidine compounds are capecitabine, floxuridine, and fluorouracil (5-FU). Said fluoropyrimidine can be administered alone or in combination with an additional agent. The additional agent may be a molecule capable of interacting with DNA, causing e.g. the inhibition of DNA synthesis and/or preventing DNA unwinding, or a treatment with monoclonal antibodies. Therapy in the context of this invention may comprise the administration of, e.g., 5-Fluorouracil, Capecitabine, Irinotecan, Oxaliplatin, Leucovorin, Cetuximab, Panitumumab, Bevacizumab, Gefitinib, Matuzumab, AEE788, HKI-272, hR3, ICR62, Gefitinib, Erlotinib, Lapatinib, EKB-569 Sorafenib, AMT2003, vemurafenib, regorafenib, ramucirumab, ipilimumab, pembrolizumab, dabrafenib, trametinib, Zviv-Aflibercept, nivolumab, avelumab, atezolizumab, pidilizumab, MSB0010718C, MEDI4736, or combinations thereof.

In the context of this invention, stage IV CRC is a cancer in which the cancer has spread to the lymph nodes or distant organs. Stage IV CRC shall be treated by surgical resection of the tumor, and a tumor treatment regimen that comprises chemotherapy and in addition the administration of, e.g., antibodies, or an endocrine therapy, or combinations thereof. Effective antibodies that can be used for the treatment of cancer, such as CRC, are, for example, bevacizumab and cetuximab.

Preferred therapeutic agents for the treatment of CRC, in particular of late stage CRC, are, in a preferred embodiment, selected from 5-Fluorouracil, Capecitabine, Irinotecan, Oxaliplatin, Leucovorin, Cetuximab, Panitumumab, Bevacizumab, Gefitinib, Matuzumab, AEE788, HKI-272, hR3, ICR62, Gefitinib, Erlotinib, Lapatinib, EKB-569, Sorafenib, AMT2003, Vemurafenib, Regorafenib, Ramucirumab, Ipilimumab, Pembrolizumab, Dabrafenib, Trametinib, Zviv-Aflibercept, Nivolumab, Avelumab, Atezolizumab, Pidilizumab, MSB0010718C, MEDI4736, or any other therapeutic agent used to treat colorectal cancer, and combinations thereof.

In a preferred embodiment the method may include a step of treatment of the subject, preferably with a therapy selected according to the method. Such steps may include surgical steps of removal of tumor mass, the application of therapeutically effective cell suspensions, such as autologous cell therapy, or the administration of therapeutic compounds, small molecules or biologies, effective for the treatment of the disease.

Yet another aspect of this invention then relates to a method for determining a blood protein marker status in a patient, preferably a cancer patient, more preferably a colorectal cancer patient, comprising the steps of:
(a) Providing a biological sample from the subject,
(b) Determining the level of at least one or more biomarker selected from the group consisting of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON3), Apolipoprotein C I (APOCi), Gelsolin (GSN), Hepatocyte growth factor like protein (MSTi), and Transferrin receptor protein 1 (TFRC), in the biological sample, and
(c) Determining the blood protein marker status from the determined levels in step (b).

A further aspect of this invention pertains to a method for evaluating the treatment success of a patient who received a cancer treatment, comprising
**(a)** Providing a biological sample from said patient who received a cancer treatment,
**(b)** Determining the level of at least one or more biomarker selected from the group consisting of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON3), Apolipoprotein C I (APOCi), Gelsolin (GSN), Hepatocyte growth factor like protein (MSTi), and Transferrin receptor protein 1 (TFRC), in the biological sample, and
**(c)** Comparing the level of said at least one biomarker as determined in (b) with a reference,
wherein a decrease or an increase of the level of at least one or more biomarker in the biological sample from said subject compared to said reference sample or reference value is an indication for the patient's response to said treatment.

In the context of the method for evaluating the treatment success of a patient who received a cancer treatment of this invention, said reference corresponds, in a preferred embodiment, to the level of said of at least one biomarker in a provided biological sample obtained from said patient before receiving said treatment in a preferred embodiment.

Yet another aspect of this invention relates to a method of treating cancer in a patient, comprising any method according to this invention, and treating and/or preventing cancer or the recurrence of cancer in the patient by administering to the subject a therapeutically effective amount of a therapeutic agent.

A further embodiment relates to a method of this invention, wherein the method further comprises the detection of the presence of blood in a stool sample of the subject, preferably by using a fecal immunochemical test (FIT). However, the skilled artisan is aware of other methods for detecting the presence of blood in a stool sample of the subject.

In another aspect of the present invention, the invention provides diagnostic kits for aiding a diagnosis of cancer, wherein the kits can be used to detect the biomarkers of the present invention. Particularly preferred is that the diagnostic kits comprise means for quantifying the level of at least one biomarker of the invention.

For example, the diagnostic kits can be used to detect any one or a combination of the at least one biomarker described above, wherein the at least one biomarker is differentially present in samples of a patient having cancer compared to a healthy patient. The kits of the invention have many applications. For example, the kits can be used to differentiate if a subject has cancer, or has a negative diagnosis, thus aiding a cancer diagnosis. In another example, the kits can be used to identify compounds that modulate expression of the biomarkers in *in vitro* cancer cells or *in vivo* animal models for cancer.

Optionally, the kit can further comprise instructions for suitable operational parameters in the form of a label or a separate insert. For example, the kit may have standard instructions informing a consumer how to wash the probe after a sample of plasma is contacted with the probe.

In another embodiment, a kit comprises (a) an antibody that specifically binds to at least one biomarker of this invention; and (b) a detection reagent. In a further embodiment, the kit may optionally comprise a standard or control information so that the test sample can be compared with the control information standard to determine if the test amount of a marker detected in a sample is a diagnostic amount consistent with a diagnosis of cancer.

Preferably the kit of the invention is a diagnostic kit for performing a method in accordance with the present invention comprising means for quantifying the level of said at least one biomarker. Preferably the kit of the invention comprises means for quantifying a biomarker selected from Alpha 1 antitrypsin (A1AT), Apolipoprotein A I (APOA1), Haptoglobin (HP), Leucine rich alpha 2 glycoprotein (LRGi), Serum paraoxonase lactonase 3 (PON3), Apolipoprotein C I (APOCi), Gelsolin (GSN), Hepatocyte growth factor like protein (MSTi), and/or Transferrin receptor protein 1 (TFRC). Such means for quantifying is, for example, at least one antibody, preferably wherein the antibody is a monoclonal antibody, such as a monoclonal antibody that specifically binds to any of the aforementioned biomarkers. Such antibodies are known in the art and are commercially available.

Thus, yet another preferred embodiment relates to a diagnostic kit of this invention, wherein said kit comprises one or more antibodies, derivatives, or antigenic fragments thereof, for the detection of any of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON3), Apolipoprotein C I (APOCi), Gelsolin (GSN), Hepatocyte growth factor like protein (MSTi), and/or Transferrin receptor protein 1 (TFRC).

A further preferred embodiment relates to a diagnostic kit that comprises a combination of at least two antibodies, derivatives, or antigenic fragments thereof, for the detection of any of:
i) A1AT,
ii) APOA1,
iii) HP,
iv) LRG1,
v) PON₃,
vi) APOC1,
vii) GSN,
viii) MST1, and
ix) TFRC.

Thus, in the context of a particularly preferred embodiment of this invention, a method and/or a kit is preferred that comprises means and/or methods for the detection of A1AT and APOA1. Further preferred is a method and/or a kit for the detection of A1AT and HP. Additionally preferred is a method and/or a kit for the detection of A1AT and LRG1. Also preferred is a method and/or a kit for the detection of A1AT and PON3*.* Further preferred is a method and/or a kit for the detection of A1AT and APOC1. Additionally preferred is a method and/or a kit for the detection of A1AT and GSN. Also preferred is a method and/or a kit for the detection of A1AT and MST1. Further preferred is a method and/or a kit for the detection of A1AT and TFRC.

In an additional embodiment of this invention, a method and/or a kit is preferred that comprises means and/or methods for the detection of APOA1 and HP. Additionally preferred is a method and/or a kit for the detection of APOA1 and LRG1. Also preferred is a method and/or a kit for the detection of APOA1 and PON3. Further preferred is a method and/or a kit for the detection of APOA1 and APOC1. Additionally preferred is a method and/or a kit for the detection of APOA1 and GSN. Also preferred is a method and/or a kit for the detection of APOA1 and MST1. Further preferred is a method and/or a kit for the detection of APOA1 and TFRC.

Also preferred is a method and/or a kit for the detection of HP and LRG1. Also preferred is a method and/or a kit for the detection of HP and PON3. Further preferred is a method and/or a kit for the detection of HP and APOC1. Additionally preferred is a method and/or a kit for the detection of HP and GSN. Also preferred is a method and/or a kit for the detection of HP and MST1. Further preferred is a method and/or a kit for the detection of HP and TFRC.

Additionally preferred is a method and/or a kit for the detection of LRG1 and PON3. Further preferred is a method and/or a kit for the detection of LRG1 and APOC1. Additionally preferred is a method and/or a kit for the detection of LRG1 and GSN. Also preferred is a method and/or a kit for the detection of LRG1 and MST1. Further preferred is a method and/or a kit for the detection of LRG1 and TFRC.

Also preferred is a method and/or a kit for the detection of PON3 and APOC1. Additionally preferred is a method and/or a kit for the detection of PON3 and GSN. Also preferred is a method and/or a kit for the detection of PON₃ and MST1. Further preferred is a method and/or a kit for the detection of PON₃ and TFRC.

Yet another preferred embodiment relates to a method and/or a kit for the detection of APOC1 and GSN. Also preferred is a method and/or a kit for the detection of APOC1 and MST1. Further preferred is a method and/or a kit for the detection of APOC1 and TFRC.

Preferred is also a method and/or a kit for the detection of GSN and MST1. Further preferred is a method and/or a kit for the detection of GSN and TFRC.

Further preferred is a method and/or a kit for the detection of MST1 and TFRC.

Yet a further preferred embodiment relates to a diagnostic kit comprising a combination of antibodies, derivatives, or antigenic fragments thereof, for the detection of i) HP, ii) LRG1, and iii) PON3*.*

In a an additional embodiment, the diagnostic kit preferably comprises a combination of antibodies, derivatives, or antigenic fragments thereof, for the detection of
i) A1AT,
ii) APOA1,
iii) HP,
iv) LRG1, and
v) PON3.

A further preferred embodiment relates to a diagnostic kit comprising a combination of antibodies, derivatives, or antigenic fragments thereof, for the detection of any of:
i) A1AT,
ii) APOA1,
iii) HP,
iv) LRG1,
v) APOC1,
vi) GSN,
vii) MST1, and
viii) TFRC.

A further aspect of this invention relates to the use of an antibody, derivative, or antigenic fragment thereof, directed to any one of the protein biomarkers selected from A1AT, APOA1, HP, LRG1, PON3, APOC1, GSN, MST1, and TFRC, in the performance of a method according to this invention.

Yet an additional aspect of this invention pertains to the use of a biomarker selected from the group consisting of A1AT, APOA1, HP, LRG1, PON₃, APOC1, GSN, MST1, and TFRC, in a screening method for a cancer therapeutic.

The terms "of the [present] invention", "in accordance with the invention", "according to the invention" and the like, as used herein are intended to refer to all aspects and embodiments of the invention described and/or claimed herein.

As used herein, the term "comprising" is to be construed as encompassing both "including" and "consisting of', both meanings being specifically intended, and hence individually disclosed embodiments in accordance with the present invention. Where used herein, "and/or" is to be taken as specific disclosure of each of the two specified features or components with or without the other. For example, "A and/or B" is to be taken as specific disclosure of each of (i) A, (ii) B and (iii) A and B, just as if each is set out individually herein. In the context of the present invention, the terms "about" and "approximately" denote an interval of accuracy that the person skilled in the art will understand to still ensure the technical effect of the feature in question. The term typically indicates deviation from the indicated numerical value by ±20%, ±15%, ±10%, and for example ±5%. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. As will be appreciated by the person of ordinary skill, the specific such deviation for a numerical value for a given technical effect will depend on the nature of the technical effect. For example, a natural or biological technical effect may generally have a larger such deviation than one for a man-made or engineering technical effect. Where an indefinite or definite article is used when referring to a singular noun, e.g. "a", "an" or "the", this includes a plural of that noun unless something else is specifically stated.

It is to be understood that application of the teachings of the present invention to a specific problem or environment, and the inclusion of variations of the present invention or additional features thereto (such as further aspects and embodiments), will be within the capabilities of one having ordinary skill in the art in light of the teachings contained herein.

Unless context dictates otherwise, the descriptions and definitions of the features set out above are not limited to any particular aspect or embodiment of the invention and apply equally to all aspects and embodiments which are described.

The present invention will now be further described in the following examples with reference to the accompanying figures, nevertheless, without being limited thereto. For the purposes of the present invention, all references, patents, and publications as cited herein are hereby incorporated by reference in their entireties.

### BRIEF DESCRIPTION OF THE FIGURES

The figures show:
**Figure 1** shows the STARD (Standards for Reporting of Diagnostic Accuracy) flow diagram of the BLITZ-Study. Abbreviations: AA- Advanced Adenoma; CRC- Colorectal Cancer; HPP- Hyperplastic Polyps; NAA- Non advanced Adenoma; NCP- Non classified polyp; SP-Serrated poly. *The exclusion criterions for selection of CRC cases were not applicable after this point.

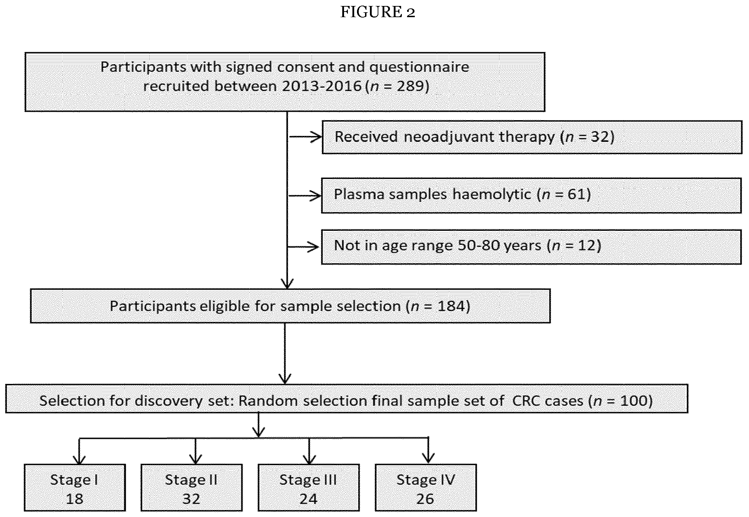

**Figure 2** shows the STARD (Standards for Reporting of Diagnostic Accuracy) flow diagram of the selection of study participants enrolled in the iDa Study. Abbreviation: CRC-Colorectal Cancer.

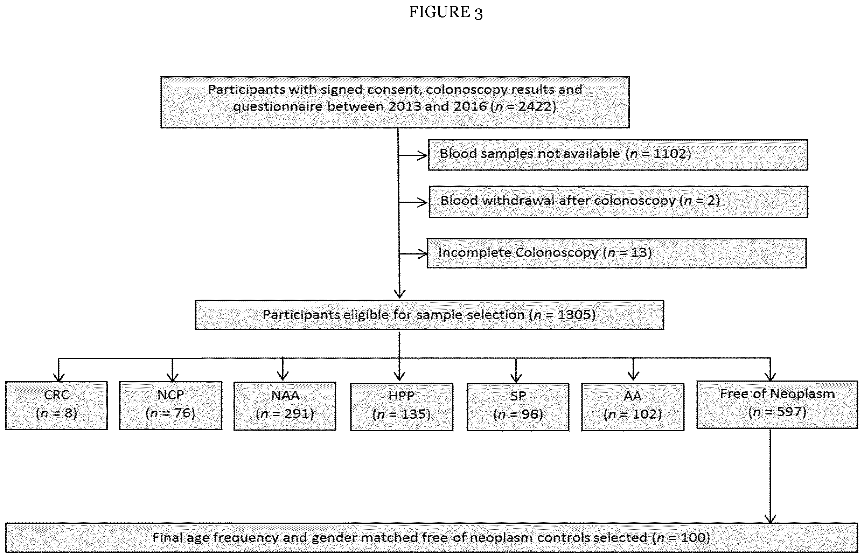

**Figure 3** shows the STARD (Standards for Reporting of Diagnostic Accuracy) flow diagram of the selection of study participants enrolled in the ASTER Study during 2013-2016. Abbreviations: AA- Advanced Adenoma; CRC- Colorectal Cancer; NAA- Non advanced Adenoma; NCP- Non classified polyp.
**Figure 4** shows the comparison of the ROC curves for detecting (all/early/late) stage CRC vs. free of neoplasm controls in discovery and validation sets. Abbreviations: AA- Advanced Adenomas; AUC- Area under the curve; CRC- Colorectal Cancer; ROC- Receiver operating characteristics.

**The Sequences show:**

SEQ ID NO: 1 >sp|P01009|A1AT_HUMAN Alpha-1-antitrypsin OS=Homo sapiens OX=9606 GN=SERPINA1 PE=1 SV=3

MPSSSWGILLLAGLCCLVPVSLAEDPQGDAAQKTDTSHHDQDHPTFNKITPNLAEFAFSLYRQL
AHQSNSTNIFFSPVSIATAFAMLSLGTKADTHDEILEGLNFNLTEIPEAQIHEGFQELLRTLNQP
DSQLQLTTGNGLFLSEGLKLVDKFLEDVKKLYHSEAFTVNFGDTEEAKKQINDYVEKGTQGKIV
DLVKELDRDTVFALVNYIFFKGKWERPFEVKDTEEEDFHVDQVTTVKVPMMKRLGMFNIQHC
KKLSSWVLLMKYLGNATAIFFLPDEGKLQHLENELTHDIITKFLENEDRRSASLHLPKLSITGTY
DLKSVLGQLGITKVFSNGADLSGVTEEAPLKLSKAVHKAVLTIDEKGTEAAGAMFLEAIPMSIPP
EVKFNKPFVFLMIEQNTKSPLFMGKVVNPTQK

SEQ ID NO: 2 >sp|P02647|APOA1_HUMAN Apolipoprotein A-I OS=Homo sapiens
OX=9606 GN=APOA1 PE=1 SV=1







SEQ ID NO: 3 >sp|P00738|HPT_HUMAN Haptoglobin OS=Homo sapiens OX=9606
GN=HP PE=1 SV=1

MSALGAVIALLLWGQLFAVDSGNDVTDIADDGCPKPPEIAHGYVEHSVRYQCKNYYKLRTEGD
GVYTLNDKKQWINKAVGDKLPECEADDGCPKPPEIAHGYVEHSVRYQCKNYYKLRTEGDGVYT

LNNEKQWINKAVGDKLPECEAVCGKPKNPANPVQRILGGHLDAKGSFPWQAKMVSHHNLTT
GATLINEQWLLTTAKNLFLNHSENATAKDIAPTLTLYVGKKQLVEIEKVVLHPNYSQVDIGLIKL
KQKVSVNERVMPICLPSKDYAEVGRVGYVSGWGRNANFKFTDHLKYVMLPVADQDQCIRHYE
GSTVPEKKTPKSPVGVQPILNEHTFCAGMSKYQEDTCYGDAGSAFAVHDLEEDTWYATGILSFD
KSCAVAEYGVYVKVTSIQDWVQKTIAEN

SEQ ID NO: 4 >sp|P02750|A2GL_HUMAN Leucine-rich alpha-2-glycoprotein
OS=Homo sapiens OX=9606 GN=LRG1 PE=1 SV=2

MSSWSRQRPKSPGGIQPHVSRTLFLLLLLAASAWGVTLSPKDCQVFRSDHGSSISCQPPAEIPGY
LPADTVHLAVEFFNLTHLPANLLQGASKLQELHLSSNGLESLSPEFLRPVPQLRVLDLTRNALT
GLPPGLFQASATLDTLVLKENQLEVLEVSWLHGLKALGHLDLSGNRLRKLPPGLLANFTLLRTL
DLGENQLETLPPDLLRGPLQLERLHLEGNKLQVLGKDLLLPQPDLRYLFLNGNKLARVAAGAF
QGLRQLDMLDLSNNSLASVPEGLWASLGQPNWDMRDGFDISGNPWICDQNLSDLYRWLQAQ
KDKMFSQNDTRCAGPEAVKGQTLLAVAKSQ

SEQ ID NO: 5 >sp|Q15166|PON3_HUMAN Serum paraoxonase/lactonase 3
OS=Homo sapiens OX=9606 GN=PON3 PE=1 SV=3

[125] MGKLVALVLLGVGLSLVGEMFLAFRERVNASREVEPVEPENCHLIEELESGSEDIDILPS
GLAFISSGLKYPGMPNFAPDEPGKIFLMDLNEQNPRAQALEISGGFDKELFNPHGISIFIDKDNT
VYLYVVNHPHMKSTVEIFKFEEQQRSLVYLKTIKHELLKSVNDIVVLGPEQFYATRDHYFTNSL
LSFFEMILDLRWTYVLFYSPREVKVVAKGFCSANGITVSADQKYVYVADVAAKNIHIMEKHDN
WDLTQLKVIQLGTLVDNLTVDPATGDILAGCHPNPMKLLNYNPEDPPGSEVLRIQNVLSEKPR
VSTVYANNGSVLQGTSVASVYHGKILIGTVFHKTLYCEL

SEQ ID NO: 6 >sp|P02654|APOC1_HUMAN Apolipoprotein C-I OS=Homo sapiens
OX=9606 GN=APOC1 PE=1 SV=1

MRLFLSLPVLVVVLSIVLEGPAPAQGTPDVSSALDKLKEFGNTLEDKARELISRIKQSELSAKMR
EWFSETFQKVKEKLKIDS

SEQ ID NO: 7 >sp|P06396|GELS_HUMAN Gelsolin OS=Homo sapiens OX=9606
GN=GSN PE=1 SV=1
















SEQ ID NO: 8 >sp|P26927|HGFL_HUMAN Hepatocyte growth factor-like protein OS=Homo sapiens OX=9606 GN=MST1 PE=1 SV=2

MGWLPLLLLLTQCLGVPGQRSPLNDFQVLRGTELQHLLHAVVPGPWQEDVADAEECAGRCGP
LMDCRAFHYNVSSHGCQLLPWTQHSPHTRLRRSGRCDLFQKKDYVRTCIMNNGVGYRGTMA
TTVGGLPCQAWSHKFPNDHKYTPTLRNGLEENFCRNPDGDPGGPWCYTTDPAVRFQSCGIKS
CREAACVWCNGEEYRGAVDRTESGRECQRWDLQHPHQHPFEPGKFLDQGLDDNYCRNPDGS
ERPWCYTTDPQIEREFCDLPRCGSEAQPRQEATTVSCFRGKGEGYRGTANTTTAGVPCQRWDA
QIPHQHRFTPEKYACKDLRENFCRNPDGSEAPWCFTLRPGMRAAFCYQIRRCTDDVRPQDCY
HGAGEQYRGTVSKTRKGVQCQRWSAETPHKPQFTFTSEPHAQLEENFCRNPDGDSHGPWCYT
MDPRTPFDYCALRRCADDQPPSILDPPDQVQFEKCGKRVDRLDQRRSKLRVVGGHPGNSPWT
VSLRNRQGQHFCGGSLVKEQWILTARQCFSSCHMPLTGYEVWLGTLFQNPQHGEPSLQRVPV
AKMVCGPSGSQLVLLKLERSVTLNQRVALICLPPEWYVVPPGTKCEIAGWGETKGTGNDTVLN
VALLNVISNQECNIKHRGRVRESEMCTEGLLAPVGACEGDYGGPLACFTHNCWVLEGIIIPNRV
CARSRWPAVFTRVSVFVDWIHKVMRLG

SEQ ID NO: 9 >sp|P02786|TFR1_HUMAN Transferrin receptor protein 1 OS=Homo sapiens OX=9606 GN=TFRC PE=1 SV=2

MMDQARSAFSNLFGGEPLSYTRFSLARQVDGDNSHVEMKLAVDEEENADNNTKANVTKPKR
CSGSICYGTIAVIVFFLIGFMIGYLGYCKGVEPKTECERLAGTESPVREEPGEDFPAARRLYWDDL
KRKLSEKLDSTDFTGTIKLLNENSYVPREAGSQKDENLALYVENQFREFKLSKVWRDQHFVKIQ
VKDSAQNSVIIVDKNGRLVYLVENPGGYVAYSKAATVTGKLVHANFGTKKDFEDLYTPVNGSIV
IVRAGKITFAEKVANAESLNAIGVLIYMDQTKFPIVNAELSFFGHAHLGTGDPYTPGFPSFNHTQ
FPPSRSSGLPNIPVQTISRAAAEKLFGNMEGDCPSDWKTDSTCRMVTSESKNVKLTVSNVLKEI
KILNIFGVIKGFVEPDHYVVVGAQRDAWGPGAAKSGVGTALLLKLAQMFSDMVLKDGFQPSRS
IIFASWSAGDFGSVGATEWLEGYLSSLHLKAFTYINLDKAVLGTSNFKVSASPLLYTLIEKTMQN
VKHPVTGQFLYQDSNWASKVEKLTLDNAAFPFLAYSGIPAVSFCFCEDTDYPYLGTTMDTYKEL
IERIPELNKVARAAAEVAGQFVIKLTHDVELNLDYERYNSQLLSFVRDLNQYRADIKEMGLSLQ
WLYSARGDFFRATSRLTTDFGNAEKTDRFVMKKLNDRVMRVEYHFLSPYVSPKESPFRHVFW
GSGSHTLPALLENLKLRKQNNGAFNETLFRNQLALATWTIQGAANALSGDVWDIDNEF

### EXAMPLES

Certain aspects and embodiments of the invention will now be illustrated by way of example and with reference to the description, figures and tables set out herein. Such examples of the methods, uses and other aspects of the present invention are representative only, and should not be taken to limit the scope of the present invention to only such representative examples.

The examples show:

### Study Design and Study Population:

The analysis was conducted in the context of the BliTz study ("Begleitende Evaluierung innovativer Testverfahren zur Darmkrebsfrüherkennung"). Briefly, BliTz is an ongoing study among participants of the German screening colonoscopy program that is offered to men and women aged 55 and older. Recruitment has been performed in 20 gastroenterology practices since end of the year 2005. Out of 9245 participants of BLITZ recruited by the end of 2016, 7107 met the inclusion criteria for this study. Among these CRC and advanced adenoma (AA), the precursors of CRC had been detected in 56 and 623 participants, respectively. In the current study, validation of protein signatures derived in the discovery set were carried out in blood samples from all 56 participants with CRC and 99 controls who were free of colorectal neoplasm. Furthermore, 99 participants with advanced adenomas (defined as adenoma with >1 cm in diameter, tubulovillous or villous components, or highgrade dysplasia) were selected. According to common practice in biomarker research, the controls free of neoplasms and AA participants were frequency matched to the CRC cases by sex and age.

The protein marker signatures were developed in a two stage design, with construction of multi-marker algorithms in a discovery set, followed by evaluation and validation of findings in an independent validation set. The independent external validation of potentially promising signatures was performed in blood samples selected from participants of screening colonoscopy collected in the BliTz study.

In the discovery set, 100 CRC cases aged 50-79 years were selected from patients recruited prior to any therapeutic measures by the iDa ("Durch **i**nnovative Testverfahren **Da**rmkrebs früher erkennen") study in hospitals in southwestern Germany between 2013 and 2016. As controls, participants of screening colonoscopy who were recruited in the ASTER ("Mit **AS**S Darm**t**umore früher **er**kennen") study in gastroenterology practices in southwestern Germany in 2013-2016 and who were found to be free of neoplasms served as controls. The ASTER study is a multicenter prospective randomized controlled trial (EudraCT No.2011-005603-32). Applying frequency matching by age and sex, the inventors randomly selected 100 controls. The iDa and ASTER studies and the use of samples for evaluation of early detection markers for CRC have been approved by the ethics committees of the Medical Faculty Heidelberg (S-489/2012 and AFmu-271/2012, for iDa and ASTER, respectively) and from the responsible state medical boards.

### Sample preparation and storage:

The blood draw was performed at first diagnosis of CRC before any therapeutic intervention for cancer in iDa and prior to colonoscopy in ASTER and BLITZ. After blood draw, Ethylenediaminetetraacetic acid (EDTA) plasma samples were transported to the laboratory while preserved in a cold transport chain, followed by centrifugation at 2000-2500 g for 10 minutes at 4°C and then stored at -80°C until picked out for the protein measurements. The laboratory staff was blind to any information regarding the study population.

Laboratory measurements: The plasma samples from the discovery and validation sets were analyzed at the Genome British Columbia Proteomics Centre, University of Victoria, BC, Canada by peptide based analysis using LC-MRM/MS for 270 unique proteins. The targeted quantitation of the 270 proteins was performed by peptide based analysis using LC-MRM/MS using assays that have been previously validated following the Clinical Proteome Tumor Analysis Consortium (CPTAC) guidelines for assay development. In brief, tryptic peptides were selected to serve as molecular surrogates for the 270 target proteins according to a series of peptide selection rules (*e.g*., unique sequence, devoid of oxidizable residues, previous detectability in plasma samples etc.). In order to compensate for the matrix-induced suppression or variability in LC-MS performance, ¹³C/¹⁵N-labeled peptide analogues were used as internal standards. All peptides were synthesized via Fmoc chemistry, purified (through RP-HPLC with subsequent assessment by MALDI-TOF-MS, and characterized via amino acid analysis, (AAA), and capillary zone electrophoresis (CZE). This quantitation of proteins was performed using paired heavy and light synthetic peptides for every protein and the unlabeled light peptide was employed for creating calibration curves. The heavy stable isotope-labeled internal standards (SIS) peptide was spiked into every plasma sample at a predetermined fixed concertation. The separate standards for calibration and normalization ensure robust and precise measurements, even at low concentrations. The peptide concentration for all protein biomarkers were reported in fmol/µl of plasma in the samples from the discovery and validation sets.

### Statistical analyses:

The data was visualized and examined with Skyline Quantitative Analysis software (version 4.1.1.18179, University of Washington). The samples were analyzed with respect to peak inspection to ensure accurate selection, integration, and uniformity (in terms of peak shape and retention time) of the SIS and natural (NAT) peptides. After defining criteria (like 1/x regression weighting, <20% deviation in the quality control level's accuracy) the standard curve was used to calculate the peptide concentration in fmol/µl of plasma.

For all samples measured across discovery and validation sets, the concentrations of several proteins were reported as zero when the abundance of NAT peptides was below the limit of quantitation and these proteins were excluded from the analyses. The concentration values of each protein biomarker were standardized by Z-score and these values were analyzed individually in both the discovery and the validation sets for detecting CRC using Wilcoxon rank sum test and correction for multiple testing by the Benjamini and Hochberg (BH) method. A prediction algorithm was constructed for each protein biomarker using logistic regression model and the diagnostic performance was evaluated by calculating areas under the ROC curves (AUCs) with 95% confidence intervals and sensitivities at cutoffs yielding 80% and 90% specificities.

For constructing multi-marker prediction algorithms markers that were found to be significant after correction for multiple testing by the BH method were considered (adjusted p-values ≤0.05). LASSO (Least absolute shrinkage and selection operator) logistic regression models with .632+ bootstrap were applied to the discovery set measurements and three prediction algorithms were derived for all (I-IV), early (I-II) and late (III-IV) stage CRC vs controls, respectively.

The three stage specific prediction algorithms were then externally evaluated and validated in the validation set comprising CRC cases and sex and age matched controls without colorectal neoplasms selected from participants of screening colonoscopy. The early stage algorithm was additionally evaluated for AA participants in comparison with controls free of neoplasms. Because the sex and age distribution of participants with AA or free of neoplasm actually differs from the corresponding distributions among CRC cases in true screening practice, observations from participants with AA and without neoplasms were weighted in the analysis in such a way that their sex and age distribution reflects the sex and age distribution of all participants with AA and without neoplasms among the participants of screening colonoscopy, respectively, in order to provide valid estimates of the algorithms' performance in a true screening setting. The diagnostic performances were evaluated by calculating AUCs and sensitivities at cutoff yielding 80% and 90% specificities.

All statistical analyses were performed with statistical software R language and environment (version 3.5.3, R core team) and p-values of 0.05 or less in two-sided testing were considered to be statistically significant.

### Example 1: Identification of 17 Biomarkers

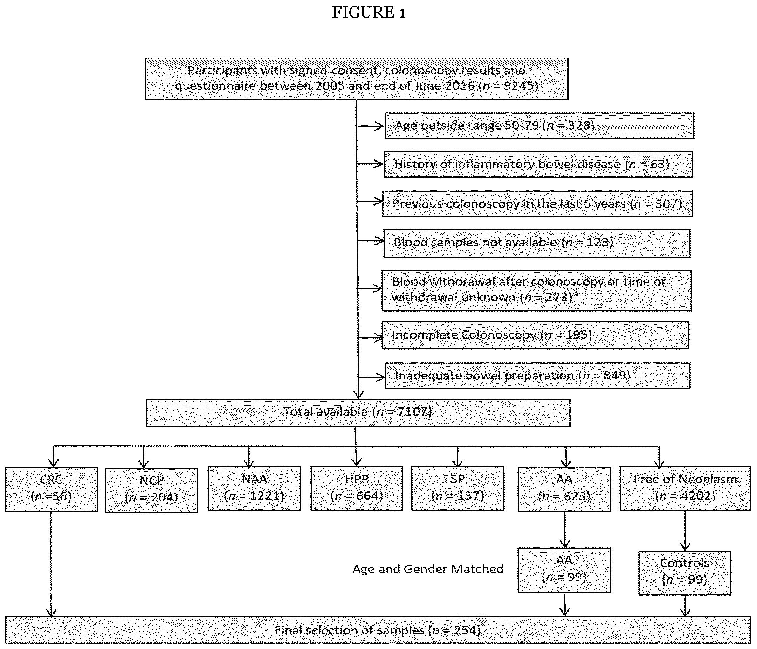

Figure 1 provides the STAandards for the Reporting of Diagnostic accuracy studies (STARD) diagrams displaying the selection of study participants enrolled in the iDa, ASTER and BLITZ study, and main characteristics of both the discovery and the validation set are shown in Table 1. The distribution of characteristics was largely similar across both sets. Males represented ≥ 60% of population in both sets, the mean age was 64 years in the discovery set and around 66 years in the validation set. The validation set included a higher proportion of stage I cancers and a lower proportion of stage IV CRC cancers than the discovery set.

**Table 1: Characteristics of the study population in both sets used for LC-MRM/MS experiments.**

| **Group** | **Discovery set** | | **Validation set** | | | **Participants of screening colonoscopy** | |
|---|---|---|---|---|---|---|---|
| | iDa (Clinical) | ASTER (Mostly Screening) | BLITZ matched set (Screening) | | | BLITZ (Screening) | |
| | CRC | Controls | CRC | AA | Controls | AA | Controls |
| Total | N (%) | N (%) | N (%) | N (%) | N (%) | N (%) | N (%) |
| | 100 | 100 | 56 | 99 | 99 | 623 | 4202 |

| **Age in years** | | | | | | | |
|---|---|---|---|---|---|---|---|
| 50-59 | 25 (25) | 29 (29) | 10 (18) | 23 (23) | 20 (20) | 237 (38) | 1916 (46) |
| 60-69 | 42 (42) | 45 (45) | 28 (50) | 46 (46) | 49 (49) | 247 (40) | 1614 (38) |
| 70-79 | 33 (33) | 26 (26) | 18 (32) | 30 (30) | 30 (30) | 139 (22) | 672 (16) |
| Mean | 64.4 | 64.0 | 66.0 | 65.4 | 65.4 | 63.3 | 61.9 |
| Median | 64.0 | 65.0 | 65.0 | 64.0 | 66.0 | 62.0 | 60.0 |
| SD | 6.9 | 7.5 | 5.8 | 6.8 | 6.9 | 5.9 | 6.5 |

| **Gender distribution** | | | | | | | |
|---|---|---|---|---|---|---|---|
| Male | 60 (60) | 60 (60) | 36 (64) | 64 (65) | 63 (64) | 393 (63) | 1808 (43) |
| Female | 40 (40) | 40 (40) | 20 (36) | 35 (35) | 36 (36) | 230 (37) | 2394 (57) |

| **Stage distribution** | | | | | | | |
|---|---|---|---|---|---|---|---|
| Stage I | 18 (18) | - | 17 (30) | - | - | - | - |
| Stage II | 32 (32) | - | 6 (11) | - | - | - | - |
| Stage III | 24 (24) | - | 26 (46) | - | - | - | - |
| Stage IV | 26 (26) | - | 7 (13) | - | - | - | - |
| Early stage (I/II) | 50 (50) | - | 23 (41) | - | - | - | - |
| Late stage (III/IV) | 50 (50) | - | 33 (59) | - | - | - | - |

| | | | | | | | |
|---|---|---|---|---|---|---|---|
| **Abbreviations:** AA- Advanced Adenoma; Ca- Cancer; CRC- Colorectal Cancer; LC-MRM/MS-Liquid chromatography-multiple reaction monitoring/mass spectrometry; N- number; SD-Standard deviation. | | | | | | | |

### Example 2: Development of a Colorectal Cancer Diagnostic Panel

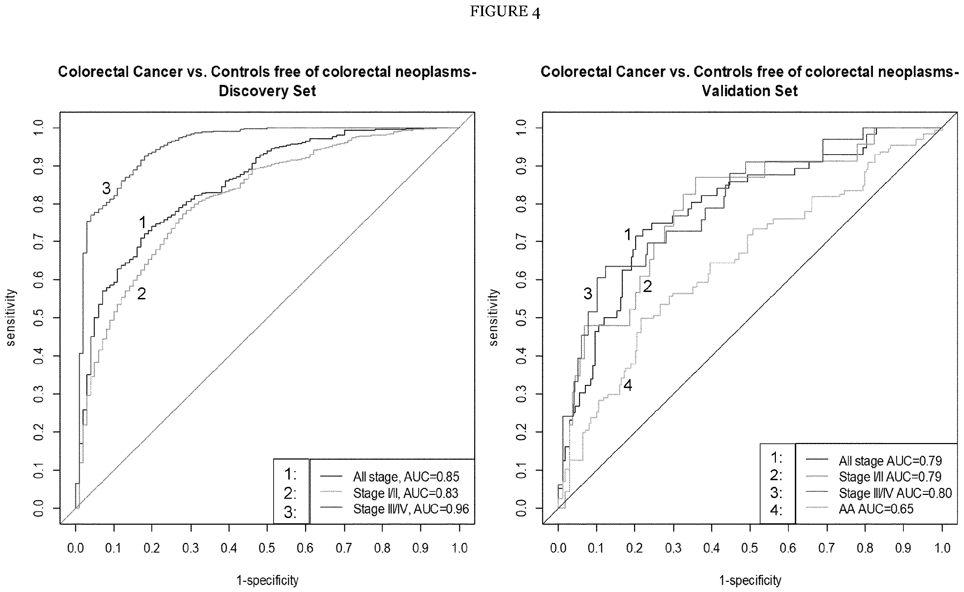

The inventors applied the LASSO Logistic regression model with .632+ bootstrap to the 83 protein biomarkers with adjusted p-values ≤0.05 in the discovery set to evaluate the performance of multimarker prediction algorithms for comparing all, early and late stages CRC to controls. As presented in Table 2 and Figure 4 the best diagnostic performances were observed for five, three and eight marker signatures for all, early and late stage CRC detection, respectively. For all stages, a five marker algorithm of proteins presented an AUC^{BS} of 0.85 and diagnostic sensitivities of 74% and 60% at cutoffs yielding 80% and 90% specificity, respectively. The proteins identified in the all stage algorithm were Alpha 1 antitrypsin (A1AT), Apolipoprotein A I (APOA1), Haptoglobin (HP), Leucine rich alpha 2 glycoprotein (LRG1) and Serum paraoxonase lactonase 3 (PON3)

**Table 2: Diagnostic performance of multimarker signatures for detecting CRC (all stages/early/late/AA) in discovery and validation sets using LC-MRM/MS.**

| ***STAGE vs. FREE OF NEOPLASM CONTROLS** | **PROTEIN MARKERS DISCOVERED IN THE SIGNATURES** | **DISCOVERY SET** | | | **VALIDATION SET (Screening population)** | | |
|---|---|---|---|---|---|---|---|
| | | AUC^{BS} | Se % at 80% Sp | Se % at 90% Sp | AUC (95% CI) | Se % at 80% Sp | Se % at 90% Sp |
| All stages CRC | A1AT + APOA1 + HP+ LRG1+ PON3 | 0.85 | 74 | 60 | 0.79 (0.70-0.86) | 68 | 46 |
| Early stages CRC | HP+ LRG1+ PON3 | 0.83 | 67 | 52 | 0.79 (0.66-0.89) | 57 | 48 |
| Late stages CRC | A1AT + APOA1+ APOC1+ GSN + HP+LRG1+ MST1+ TFRC | 0.96 | 93 | 82 | 0.80 (0.70-0.89) | 64 | 52 |
| AA | HP+ LRG1+ PON3 | - | - | - | 0.65 (0.56-0.73) | 41 | 25 |

| | | | | | | | |
|---|---|---|---|---|---|---|---|
| **Abbreviations:** AA- Advanced Adenomas; AUC- Area under the Receiver Operating Curve; AUC^{BS}- .632+ bootstrap estimates of AUC; CRC- Colorectal Cancer; 95% CI- 95 % Confidence Interval; LC-MRM/MS- Liquid chromatography-multiple reaction monitoring/mass spectrometry; Se- Sensitivity; Sp- Specificity; A1AT- Alpha 1 antitrypsin; APOA1-Apolipoprotein A I; APOC1- Apolipoprotein C I; GSN- Gelsolin; HP- Haptoglobin; LRGi-Leucine rich alpha 2 glycoprotein; MSTi- Hepatocyte growth factor like protein; PON₃- Serum paraoxonase lactonase 3; TFRC- Transferrin receptor protein 1. | | | | | | | |

The inventors identified three different multi-marker signatures and a total of nine different proteins in the three prediction models.

Three different signatures for all, early and late stages CRC consisting of five, three and eight protein markers were obtained in the discovery set with areas under the curves (AUCs) after .632+ bootstrap adjustment of 0.90, 0.83 and 0.96, respectively. External validation in the representative screening population yielded AUCs of 0.79 (95%CI, 0.70-0.86), 0.79 (95%CI, 0.66-0.89) and 0.80 (95%CI, 0.70-0.89) for all, early and late stage CRCs, respectively. The three marker early stage algorithm yielded an AUC of 0.65 (95%CI, 0.56-0.73) for detection of AA in the validation set.

A five marker signature with an AUC^{BS} of 0.90 in the discovery set and of 0.79 (95%CI, 0.70-0.86) in the validation set best detected all stage CRC.

A three marker signature with an AUC^{BS} of 0.83 in the discovery set and of 0.83 in the discovery set and of 0.79 (95%CI, 0.66-0.89) in the validation set best detected early stage CRC.

An eight marker signature with an AUC^{BS} of 0.96 in the discovery set and of 0.83 in the discovery set and of 0.80 (95%CI, 0.70-0.89) in the validation set best detected late stage CRC.

As shown in Table 2, for the early stage CRC cases vs controls comparison the algorithm consisting of three proteins HP, LRG1 and PON₃, was identified in the discovery set, with an AUC^{BS} of 0.83 and sensitivities of 67% and 52% at cutoffs yielding 80% and 90% specificity, respectively. For late stage CRC detection, an eight marker signature was identified in the discovery set, with an AUC^{BS} of 0.96 and sensitivities of 93% and 82% at cutoffs yielding 80% and 90% specificity, respectively. Four of the proteins (A1AT, APOA1, HP and LRG1) were identical with the algorithm identified for the all stages, the other four were Apolipoprotein C I (APOC1), Gelsolin (GSN), Hepatocyte growth factor like protein (MST1) and Transferrin receptor protein 1 (TFRC).

### Example 3: Validation of the Diagnostic Panel of five, three and eight Biomarkers in the Diagnosis of CRC and AA

Validation of the five, three and eight marker algorithms yielded AUCs of 0.79 (95%CI, 0.70-0.86), 0.79 (95%CI, 0.66-0.89) and 0.80(95%CI, 0.70-0.89) for all, early and late stage CRC detection, respectively, in the validation sample from the screening population. Sensitivities of 68%, 57% and 64% at cut-offs yielding 80% specificity and of 46%, 48% and 52% at cut-offs yielding 90% specificity were observed for all, early and late stage CRC detection, respectively. When the three marker algorithm for early stages was applied to screening participants with AA, an AUC of 0.65 (95%CI, 0.56-0.73) and sensitivities of 41% and 25% at cutoffs yielding 80% and 90% specificity were obtained.

The molecular functions of the nine protein biomarkers that were identified into three different signatures as extracted from the UniProt database are presented in the Table 3. These proteins have a wide variety of molecular functions from protease inhibitor to binding. The protein biomarkers seem to be involved in a wide range of biological processes like angiogenesis, adhesion, homeostasis, and chemotaxis.

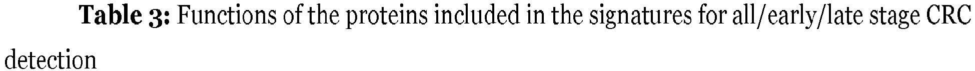

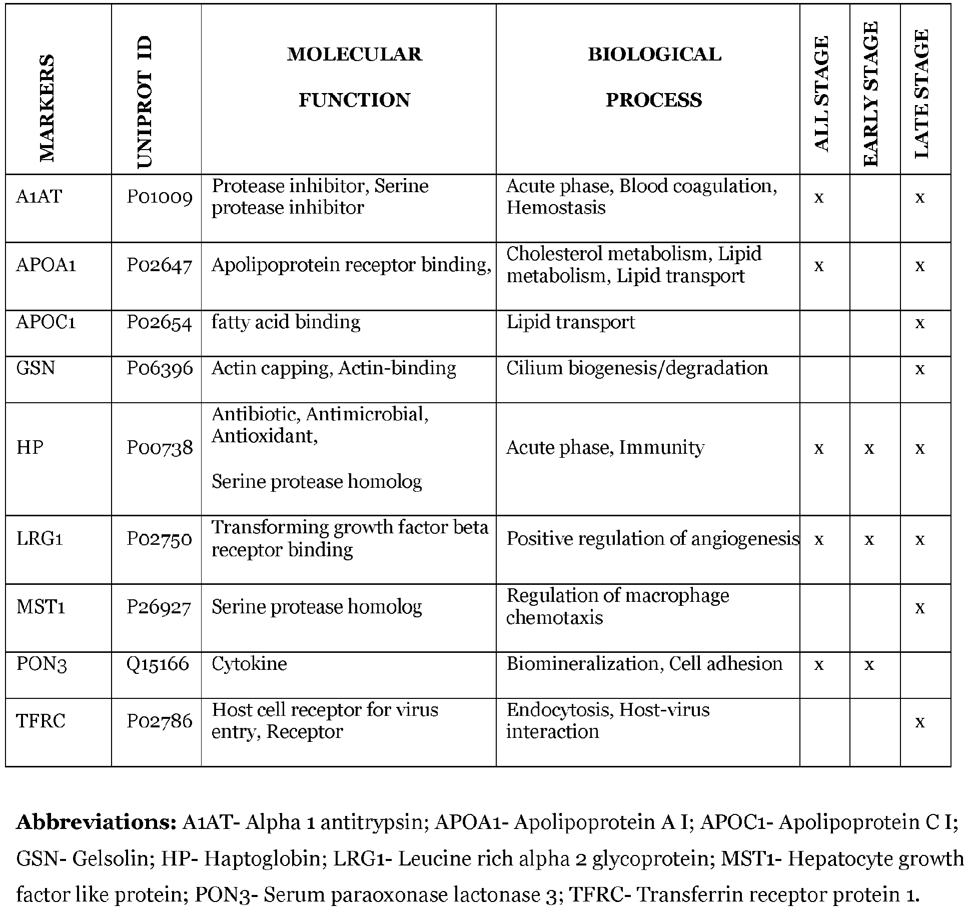

### Example 4: Prediction algorithms

**Table 4. The algorithms identified from the discovery set and evaluated on validation set for (all/early/late) stage CRC and AA detection.**

| ***STAGE vs. FREE OF NEOPLASM CONTROLS** | **ALGORITHMS** |
|---|---|
| **All stages CRC** | 0.0192 +**HP***0.5624 +**LRG1***0.0481 +**A1AT***0.0332 +**APOA1***-0.0473 +**PON3***-0.0294 |
| **Early stages CRC** | -0.4681 +**HP***0.4477 +**LRG1***0.3863 +**PON3***-0.0892 |
| **Late stages CRC** | -0.7199 +**HP***0.4863 +**LRG1***0.0237 +**A1AT***0.2799 +**GSN***-0.2542 +**APOA1***0.1589 +**MST1***0.1596 +**TFRC***0.1023 +**APOC1***-0.0734 |
| **AA** | -0.4681 +**HP***0.4477 +**LRG1***0.3863 +**PON3**"-0.0892 |

| | |
|---|---|
| **Abbreviations:** AA- Advanced Adenomas; CRC- Colorectal Cancer. All proteins abbreviations: A1AT- Alpha 1 antitrypsin; APOA1- Apolipoprotein A I; APOC1- Apolipoprotein C I; GSN-Gelsolin; HP- Haptoglobin; LRGi- Leucine rich alpha 2 glycoprotein; MSTi- Hepatocyte growth factor like protein; PON₃- Serum paraoxonase lactonase 3; TFRC- Transferrin receptor protein 1. | |

## Claims

1. **A method for the diagnosis, prognosis, stratification and/or monitoring of a therapy, of a cancer disease in a subject,** comprising the steps of:
**(a)** Providing a biological sample from the subject,
**(b)** Determining the level of at least one or more biomarker selected from the group consisting of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON3), Apolipoprotein C I (APOC1), Gelsolin (GSN), Hepatocyte growth factor like protein (MSTi), and Transferrin receptor protein 1 (TFRC), in the biological sample,
wherein a differential level of the at least one or more biomarker in the biological sample from the subject as determined in step **(b)** compared to a healthy control or reference value is indicative for the presence of a cancer disease in the subject.

2. The method according to claim 1, wherein step **(b)** comprises determining the level of at least HP, LRG1, and PON₃, in the biological sample, preferably wherein the cancer disease is colorectal cancer, and/or advanced adenoma, more preferably wherein the colorectal cancer is early stage o/I/II colorectal cancer.

3. The method according to claim 1, wherein step **(b)** comprises determining the level of at least A1AT, APOA1, HP, LRG1, and PON3, in the biological sample, preferably wherein the cancer disease is colorectal cancer.

4. The method according to claim 1, wherein step **(b)** comprises determining the level of at least A1AT, APOA1, HP, LRG1, APOC1, GSN, MST1, and TFRC, in the biological sample, preferably wherein the cancer disease is colorectal cancer, more preferably wherein the colorectal cancer is late stage III/IV colorectal cancer.

5. The method according to any one of claims 1 to 4, wherein the biological sample is a sample of a subject comprising a tissue sample or a body liquid sample, such as a blood sample, a serum sample, a plasma sample, a sample of a group of cells from a tumor, a tumor tissue, a urine sample, a lymph fluid sample, a pleural fluid sample, or a brain liquor sample, preferably wherein the biological sample is a blood sample, such as a plasma sample.

6. The method according to any one of claims 1 to 5, wherein the biomarker is a protein biomarker, and wherein the determining the level of the at least one or more biomarker involves determining the level of a protein of the at least one or more biomarker in the biological sample.

7. The method according to any one of claims 1 to 6, wherein the method is a screening method for establishing a first diagnosis of cancer in the subject.

8. The method according to any one of claims 1 to 7, wherein the cancer is colorectal cancer, advanced adenoma, pancreatic cancer, gastric cancer, breast cancer, lung cancer, prostate cancer, hepatocellular cancer, cervical cancer, ovarian cancer, liver cancer, bladder cancer, cancer of the urinary tract, thyroid cancer, renal cancer, carcinoma, melanoma, leukemia, or brain cancer, preferably wherein the cancer is colorectal cancer, and/or advanced adenoma, optionally wherein said colorectal cancer is early stage o/I/II colorectal cancer, or late stage III/IV colorectal cancer.

9. The method according to any one of claims 1 to 8, wherein a differential level of a biomarker selected from A1AT, APOA1, HP, LRG1, PON₃, APOC1, GSN, MST1, and TFRC is a higher level.

10. The method according to any one of claims 1 to 8, wherein a differential level of a biomarker selected from A1AT, APOA1, HP, LRG1, PON3, APOC1, GSN, MST1, and TFRC is a lower level.

11. The method according to any one of claims 1 to 11, wherein the biomarker is detected using one or more binding agents or antigen binding proteins, such as antibodies, preferably wherein the biomarker is detected by western blot, ELISA, Proximity Extension Assay, or mass-spectrometrically.

12. **A method of selecting a therapy for treating colorectal cancer in a subject,** said method comprising the steps of:
**(a)** Obtaining a biological sample from the subject,
**(b)** Determining the level of at least one or more biomarker selected from the group consisting of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON3), Apolipoprotein C I (APOC1), Gelsolin (GSN), Hepatocyte growth factor like protein (MST1), and Transferrin receptor protein **1** (TFRC), in the biological sample, and
**(c)** Deciding in favor or against a therapy,
wherein, if **(i)** no differential level of the at least one or more biomarker in the biological sample from the subject as determined in step **(b)** compared to a healthy control or reference value is determined, it is decided against said therapy in step **(c)**, and wherein if **(ii)** a differential level of the at least one or more biomarker in the biological sample from the subject as determined in step **(b)** compared to a healthy control or reference value is determined to be indicative for early stage o/I/II colorectal cancer, it is decided in favor of a surgical type of therapy in step **(c)**, thereby treating the colorectal cancer in the subject, and if **(iii)** a differential level of the at least one or more biomarker in the biological sample from the subject as determined in step **(b)** compared to a healthy control or reference value is determined to be indicative for late stage III/IV colorectal cancer, it is decided in favor of a surgical type of therapy, and/or a non-surgical type of therapy comprising a therapeutic agent in step **(c)**, thereby treating the colorectal cancer in the subject.

13. **A method for evaluating the treatment success of a patient who received a cancer treatment**, comprising
**(a)** Providing a biological sample from said patient who received a cancer treatment,
**(b)** Determining the level of at least one or more biomarker selected from the group consisting of Alpha 1 antitrypsin (A1AT), Apolipoprotein A I (APOA1), Haptoglobin (HP), Leucine rich alpha 2 glycoprotein (LRGi), Serum paraoxonase lactonase 3 (PON₃), Apolipoprotein C I (APOCi), Gelsolin (GSN), Hepatocyte growth factor like protein (MSTi), and Transferrin receptor protein 1 (TFRC), in the biological sample, and
**(c)** Comparing the level of said at least one biomarker as determined in **(b)** with a reference,
wherein a decrease or an increase of the level of at least one or more biomarker in the biological sample from said subject compared to said reference sample or reference value is an indication for the patient's response to said treatment.

14. The method according to claim 13, wherein said reference corresponds to the level of said of at least one biomarker in a provided biological sample obtained from said patient before receiving said treatment.

15. The method according to any one of the preceding claims, wherein said subject is a mammal, such as a mouse, a rat, a guinea pig, a rabbit, a cat, a dog, a monkey, or a human, preferably a human, such as a human patient, more preferably a human patient suffering from a disease, such as cancer, and in need of a therapy, most preferably a human patient suffering from colorectal cancer and in need of a therapy.

16. **A diagnostic kit** for performing a method according to any one of claims 1 to 15, the kit comprising a combination of at least two antibodies, derivatives, or antigenic fragments thereof, for the detection of any of:
**i)** A1AT,
**ii)** APOA1,
**iii)** HP,
**iv)** LRG1,
**v)** PON3,
**vi)** APOC1,
**vii)** GSN,
**viii)** MST1, and
**ix)** TFRC.


SEQUENCE LISTING

<110>  Deutsches Krebsforschungszentrum Stiftung des öffentlichen
       Rechts

<120>  BIOMARKER PANEL FOR DIAGNOSING COLORECTAL CANCER

<130>  D31706EP

<160>  9

<170>  PatentIn version 3.5

<210>  1
<211>  417
<212>  PRT
<213>  Homo sapiens

<400>  1

Met Pro Ser Ser Ser Trp Gly Ile Leu Leu Leu Ala Gly Leu Cys Cys
1               5                   10                  15

Leu Val Pro Val Ser Leu Ala Glu Asp Pro Gln Gly Asp Ala Ala Gln
            20                  25                  30

Lys Thr Asp Thr Ser His His Asp Gln Asp His Pro Thr Phe Asn Lys
        35                  40                  45

Ile Thr Pro Asn Leu Ala Glu Phe Ala Phe Ser Leu Tyr Arg Gln Leu
    50                  55                  60

Ala His Gln Ser Asn Ser Thr Asn Ile Phe Phe Ser Pro Val Ser Ile
65                  70                  75                  80

Ala Thr Ala Phe Ala Met Leu Ser Leu Gly Thr Lys Ala Asp Thr His
                85                  90                  95

Asp Glu Ile Leu Glu Gly Leu Asn Phe Asn Leu Thr Glu Ile Pro Glu
            100                 105                 110

Ala Gln Ile His Glu Gly Phe Gln Glu Leu Leu Arg Thr Leu Asn Gln
        115                 120                 125

Pro Asp Ser Gln Leu Gln Leu Thr Thr Gly Asn Gly Leu Phe Leu Ser
    130                 135                 140

Glu Gly Leu Lys Leu Val Asp Lys Phe Leu Glu Asp Val Lys Lys Leu
145                 150                 155                 160

Tyr His Ser Glu Ala Phe Thr Val Asn Phe Gly Asp Thr Glu Glu Ala
                165                 170                 175

Lys Lys Gln Ile Asn Asp Tyr Val Glu Lys Gly Thr Gln Gly Lys Ile
            180                 185                 190

Val Asp Leu Val Lys Glu Leu Asp Arg Asp Thr Val Phe Ala Leu Val
        195                 200                 205

Asn Tyr Ile Phe Phe Lys Gly Lys Trp Glu Arg Pro Phe Glu Val Lys
    210                 215                 220

Asp Thr Glu Glu Glu Asp Phe His Val Asp Gln Val Thr Thr Val Lys
225                 230                 235                 240

Val Pro Met Met Lys Arg Leu Gly Met Phe Asn Ile Gln His Cys Lys
                245                 250                 255

Lys Leu Ser Ser Trp Val Leu Leu Met Lys Tyr Leu Gly Asn Ala Thr
            260                 265                 270

Ala Ile Phe Phe Leu Pro Asp Glu Gly Lys Leu Gln His Leu Glu Asn
        275                 280                 285

Glu Leu Thr His Asp Ile Ile Thr Lys Phe Leu Glu Asn Glu Asp Arg
    290                 295                 300

Arg Ser Ala Ser Leu His Leu Pro Lys Leu Ser Ile Thr Gly Thr Tyr
305                 310                 315                 320

Asp Leu Lys Ser Val Leu Gly Gln Leu Gly Ile Thr Lys Val Phe Ser
                325                 330                 335

Asn Gly Ala Asp Leu Ser Gly Val Thr Glu Glu Ala Pro Leu Lys Leu
            340                 345                 350

Ser Lys Ala Val His Lys Ala Val Leu Thr Ile Asp Glu Lys Gly Thr
        355                 360                 365

Glu Ala Ala Gly Ala Met Phe Leu Glu Ala Ile Pro Met Ser Ile Pro
    370                 375                 380

Pro Glu Val Lys Phe Asn Lys Pro Phe Val Phe Leu Met Ile Glu Gln
385                 390                 395                 400

Asn Thr Lys Ser Pro Leu Phe Met Gly Lys Val Val Asn Pro Thr Gln
                405                 410                 415

Lys

<210>  2
<211>  267
<212>  PRT
<213>  Homo sapiens

<400>  2

<210>  3
<211>  406
<212>  PRT
<213>  Homo sapiens

<400>  3

Met Ser Ala Leu Gly Ala Val Ile Ala Leu Leu Leu Trp Gly Gln Leu
1               5                   10                  15

Phe Ala Val Asp Ser Gly Asn Asp Val Thr Asp Ile Ala Asp Asp Gly
            20                  25                  30

Cys Pro Lys Pro Pro Glu Ile Ala His Gly Tyr Val Glu His Ser Val
        35                  40                  45

Arg Tyr Gln Cys Lys Asn Tyr Tyr Lys Leu Arg Thr Glu Gly Asp Gly
    50                  55                  60

Val Tyr Thr Leu Asn Asp Lys Lys Gln Trp Ile Asn Lys Ala Val Gly
65                  70                  75                  80

Asp Lys Leu Pro Glu Cys Glu Ala Asp Asp Gly Cys Pro Lys Pro Pro
                85                  90                  95

Glu Ile Ala His Gly Tyr Val Glu His Ser Val Arg Tyr Gln Cys Lys
            100                 105                 110

Asn Tyr Tyr Lys Leu Arg Thr Glu Gly Asp Gly Val Tyr Thr Leu Asn
        115                 120                 125

Asn Glu Lys Gln Trp Ile Asn Lys Ala Val Gly Asp Lys Leu Pro Glu
    130                 135                 140

Cys Glu Ala Val Cys Gly Lys Pro Lys Asn Pro Ala Asn Pro Val Gln
145                 150                 155                 160

Arg Ile Leu Gly Gly His Leu Asp Ala Lys Gly Ser Phe Pro Trp Gln
                165                 170                 175

Ala Lys Met Val Ser His His Asn Leu Thr Thr Gly Ala Thr Leu Ile
                180                 185                 190

Asn Glu Gln Trp Leu Leu Thr Thr Ala Lys Asn Leu Phe Leu Asn His
            195                 200                 205

Ser Glu Asn Ala Thr Ala Lys Asp Ile Ala Pro Thr Leu Thr Leu Tyr
    210                 215                 220

Val Gly Lys Lys Gln Leu Val Glu Ile Glu Lys Val Val Leu His Pro
225                 230                 235                 240

Asn Tyr Ser Gln Val Asp Ile Gly Leu Ile Lys Leu Lys Gln Lys Val
                245                 250                 255

Ser Val Asn Glu Arg Val Met Pro Ile Cys Leu Pro Ser Lys Asp Tyr
            260                 265                 270

Ala Glu Val Gly Arg Val Gly Tyr Val Ser Gly Trp Gly Arg Asn Ala
        275                 280                 285

Asn Phe Lys Phe Thr Asp His Leu Lys Tyr Val Met Leu Pro Val Ala
    290                 295                 300

Asp Gln Asp Gln Cys Ile Arg His Tyr Glu Gly Ser Thr Val Pro Glu
305                 310                 315                 320

Lys Lys Thr Pro Lys Ser Pro Val Gly Val Gln Pro Ile Leu Asn Glu
                325                 330                 335

His Thr Phe Cys Ala Gly Met Ser Lys Tyr Gln Glu Asp Thr Cys Tyr
            340                 345                 350

Gly Asp Ala Gly Ser Ala Phe Ala Val His Asp Leu Glu Glu Asp Thr
        355                 360                 365

Trp Tyr Ala Thr Gly Ile Leu Ser Phe Asp Lys Ser Cys Ala Val Ala
    370                 375                 380

Glu Tyr Gly Val Tyr Val Lys Val Thr Ser Ile Gln Asp Trp Val Gln
385                 390                 395                 400

Lys Thr Ile Ala Glu Asn
                405

<210>  4

<211>  347
<212>  PRT
<213>  Homo sapiens

<400>  4

Met Ser Ser Trp Ser Arg Gln Arg Pro Lys Ser Pro Gly Gly Ile Gln
1               5                   10                  15

Pro His Val Ser Arg Thr Leu Phe Leu Leu Leu Leu Leu Ala Ala Ser
            20                  25                  30

Ala Trp Gly Val Thr Leu Ser Pro Lys Asp Cys Gln Val Phe Arg Ser
        35                  40                  45

Asp His Gly Ser Ser Ile Ser Cys Gln Pro Pro Ala Glu Ile Pro Gly
    50                  55                  60

Tyr Leu Pro Ala Asp Thr Val His Leu Ala Val Glu Phe Phe Asn Leu
65                  70                  75                  80

Thr His Leu Pro Ala Asn Leu Leu Gln Gly Ala Ser Lys Leu Gln Glu
                85                  90                  95

Leu His Leu Ser Ser Asn Gly Leu Glu Ser Leu Ser Pro Glu Phe Leu
            100                 105                 110

Arg Pro Val Pro Gln Leu Arg Val Leu Asp Leu Thr Arg Asn Ala Leu
        115                 120                 125

Thr Gly Leu Pro Pro Gly Leu Phe Gln Ala Ser Ala Thr Leu Asp Thr
    130                 135                 140

Leu Val Leu Lys Glu Asn Gln Leu Glu Val Leu Glu Val Ser Trp Leu
145                 150                 155                 160

His Gly Leu Lys Ala Leu Gly His Leu Asp Leu Ser Gly Asn Arg Leu
                165                 170                 175

Arg Lys Leu Pro Pro Gly Leu Leu Ala Asn Phe Thr Leu Leu Arg Thr
            180                 185                 190

Leu Asp Leu Gly Glu Asn Gln Leu Glu Thr Leu Pro Pro Asp Leu Leu
        195                 200                 205

Arg Gly Pro Leu Gln Leu Glu Arg Leu His Leu Glu Gly Asn Lys Leu
    210                 215                 220

Gln Val Leu Gly Lys Asp Leu Leu Leu Pro Gln Pro Asp Leu Arg Tyr

225                 230                 235                 240

Leu Phe Leu Asn Gly Asn Lys Leu Ala Arg Val Ala Ala Gly Ala Phe
                245                 250                 255

Gln Gly Leu Arg Gln Leu Asp Met Leu Asp Leu Ser Asn Asn Ser Leu
            260                 265                 270

Ala Ser Val Pro Glu Gly Leu Trp Ala Ser Leu Gly Gln Pro Asn Trp
        275                 280                 285

Asp Met Arg Asp Gly Phe Asp Ile Ser Gly Asn Pro Trp Ile Cys Asp
    290                 295                 300

Gln Asn Leu Ser Asp Leu Tyr Arg Trp Leu Gln Ala Gln Lys Asp Lys
305                 310                 315                 320

Met Phe Ser Gln Asn Asp Thr Arg Cys Ala Gly Pro Glu Ala Val Lys
                325                 330                 335

Gly Gln Thr Leu Leu Ala Val Ala Lys Ser Gln
            340                 345

<210>  5
<211>  354
<212>  PRT
<213>  Homo sapiens

<400>  5

Met Gly Lys Leu Val Ala Leu Val Leu Leu Gly Val Gly Leu Ser Leu
1               5                   10                  15

Val Gly Glu Met Phe Leu Ala Phe Arg Glu Arg Val Asn Ala Ser Arg
            20                  25                  30

Glu Val Glu Pro Val Glu Pro Glu Asn Cys His Leu Ile Glu Glu Leu
        35                  40                  45

Glu Ser Gly Ser Glu Asp Ile Asp Ile Leu Pro Ser Gly Leu Ala Phe
    50                  55                  60

Ile Ser Ser Gly Leu Lys Tyr Pro Gly Met Pro Asn Phe Ala Pro Asp
65                  70                  75                  80

Glu Pro Gly Lys Ile Phe Leu Met Asp Leu Asn Glu Gln Asn Pro Arg
                85                  90                  95

Ala Gln Ala Leu Glu Ile Ser Gly Gly Phe Asp Lys Glu Leu Phe Asn

            100                 105                 110

Pro His Gly Ile Ser Ile Phe Ile Asp Lys Asp Asn Thr Val Tyr Leu
        115                 120                 125

Tyr Val Val Asn His Pro His Met Lys Ser Thr Val Glu Ile Phe Lys
    130                 135                 140

Phe Glu Glu Gln Gln Arg Ser Leu Val Tyr Leu Lys Thr Ile Lys His
145                 150                 155                 160

Glu Leu Leu Lys Ser Val Asn Asp Ile Val Val Leu Gly Pro Glu Gln
                165                 170                 175

Phe Tyr Ala Thr Arg Asp His Tyr Phe Thr Asn Ser Leu Leu Ser Phe
            180                 185                 190

Phe Glu Met Ile Leu Asp Leu Arg Trp Thr Tyr Val Leu Phe Tyr Ser
        195                 200                 205

Pro Arg Glu Val Lys Val Val Ala Lys Gly Phe Cys Ser Ala Asn Gly
    210                 215                 220

Ile Thr Val Ser Ala Asp Gln Lys Tyr Val Tyr Val Ala Asp Val Ala
225                 230                 235                 240

Ala Lys Asn Ile His Ile Met Glu Lys His Asp Asn Trp Asp Leu Thr
                245                 250                 255

Gln Leu Lys Val Ile Gln Leu Gly Thr Leu Val Asp Asn Leu Thr Val
            260                 265                 270

Asp Pro Ala Thr Gly Asp Ile Leu Ala Gly Cys His Pro Asn Pro Met
        275                 280                 285

Lys Leu Leu Asn Tyr Asn Pro Glu Asp Pro Pro Gly Ser Glu Val Leu
    290                 295                 300

Arg Ile Gln Asn Val Leu Ser Glu Lys Pro Arg Val Ser Thr Val Tyr
305                 310                 315                 320

Ala Asn Asn Gly Ser Val Leu Gln Gly Thr Ser Val Ala Ser Val Tyr
                325                 330                 335

His Gly Lys Ile Leu Ile Gly Thr Val Phe His Lys Thr Leu Tyr Cys
            340                 345                 350

Glu Leu

<210> 6
<211> 83
<212> PRT
<213> Homo sapiens

<400> 6

Met Arg Leu Phe Leu Ser Leu Pro Val Leu Val Val Val Leu Ser Ile
1               5                   10                  15

Val Leu Glu Gly Pro Ala Pro Ala Gln Gly Thr Pro Asp Val Ser Ser
            20                  25                  30

Ala Leu Asp Lys Leu Lys Glu Phe Gly Asn Thr Leu Glu Asp Lys Ala
        35                  40                  45

Arg Glu Leu Ile Ser Arg Ile Lys Gln Ser Glu Leu Ser Ala Lys Met
    50                  55                  60

Arg Glu Trp Phe Ser Glu Thr Phe Gln Lys Val Lys Glu Lys Leu Lys
65                  70                  75                  80

Ile Asp Ser

<210> 7
<211> 782
<212> PRT
<213> Homo sapiens

<400> 7

<210> 8
<211> 711
<212> PRT
<213> Homo sapiens

<400> 8

Met Gly Trp Leu Pro Leu Leu Leu Leu Leu Thr Gln Cys Leu Gly Val
1               5                   10                  15

Pro Gly Gln Arg Ser Pro Leu Asn Asp Phe Gln Val Leu Arg Gly Thr
            20                  25                  30

Glu Leu Gln His Leu Leu His Ala Val Val Pro Gly Pro Trp Gln Glu
        35                  40                  45

Asp Val Ala Asp Ala Glu Glu Cys Ala Gly Arg Cys Gly Pro Leu Met
    50                  55                  60

Asp Cys Arg Ala Phe His Tyr Asn Val Ser Ser His Gly Cys Gln Leu
65                  70                  75                  80

Leu Pro Trp Thr Gln His Ser Pro His Thr Arg Leu Arg Arg Ser Gly
                85                  90                  95

Arg Cys Asp Leu Phe Gln Lys Lys Asp Tyr Val Arg Thr Cys Ile Met
            100                 105                 110

Asn Asn Gly Val Gly Tyr Arg Gly Thr Met Ala Thr Thr Val Gly Gly
        115                 120                 125

Leu Pro Cys Gln Ala Trp Ser His Lys Phe Pro Asn Asp His Lys Tyr
    130                 135                 140

Thr Pro Thr Leu Arg Asn Gly Leu Glu Glu Asn Phe Cys Arg Asn Pro
145                 150                 155                 160

Asp Gly Asp Pro Gly Gly Pro Trp Cys Tyr Thr Thr Asp Pro Ala Val
                165                 170                 175

Arg Phe Gln Ser Cys Gly Ile Lys Ser Cys Arg Glu Ala Ala Cys Val
            180                 185                 190

Trp Cys Asn Gly Glu Glu Tyr Arg Gly Ala Val Asp Arg Thr Glu Ser
        195                 200                 205

Gly Arg Glu Cys Gln Arg Trp Asp Leu Gln His Pro His Gln His Pro
    210                 215                 220

Phe Glu Pro Gly Lys Phe Leu Asp Gln Gly Leu Asp Asp Asn Tyr Cys
225                 230                 235                 240

Arg Asn Pro Asp Gly Ser Glu Arg Pro Trp Cys Tyr Thr Thr Asp Pro
                245                 250                 255

Gln Ile Glu Arg Glu Phe Cys Asp Leu Pro Arg Cys Gly Ser Glu Ala
            260                 265                 270

Gln Pro Arg Gln Glu Ala Thr Thr Val Ser Cys Phe Arg Gly Lys Gly
        275                 280                 285

Glu Gly Tyr Arg Gly Thr Ala Asn Thr Thr Thr Ala Gly Val Pro Cys
    290                 295                 300

Gln Arg Trp Asp Ala Gln Ile Pro His Gln His Arg Phe Thr Pro Glu
305                 310                 315                 320

Lys Tyr Ala Cys Lys Asp Leu Arg Glu Asn Phe Cys Arg Asn Pro Asp
                325                 330                 335

Gly Ser Glu Ala Pro Trp Cys Phe Thr Leu Arg Pro Gly Met Arg Ala
            340                 345                 350

Ala Phe Cys Tyr Gln Ile Arg Arg Cys Thr Asp Asp Val Arg Pro Gln
        355                 360                 365

Asp Cys Tyr His Gly Ala Gly Glu Gln Tyr Arg Gly Thr Val Ser Lys
    370                 375                 380

Thr Arg Lys Gly Val Gln Cys Gln Arg Trp Ser Ala Glu Thr Pro His
385                 390                 395                 400

Lys Pro Gln Phe Thr Phe Thr Ser Glu Pro His Ala Gln Leu Glu Glu
                405                 410                 415

Asn Phe Cys Arg Asn Pro Asp Gly Asp Ser His Gly Pro Trp Cys Tyr
            420                 425                 430

Thr Met Asp Pro Arg Thr Pro Phe Asp Tyr Cys Ala Leu Arg Arg Cys
        435                 440                 445

Ala Asp Asp Gln Pro Pro Ser Ile Leu Asp Pro Pro Asp Gln Val Gln
    450                 455                 460

Phe Glu Lys Cys Gly Lys Arg Val Asp Arg Leu Asp Gln Arg Arg Ser
465                 470                 475                 480

Lys Leu Arg Val Val Gly Gly His Pro Gly Asn Ser Pro Trp Thr Val
                485                 490                 495

Ser Leu Arg Asn Arg Gln Gly Gln His Phe Cys Gly Gly Ser Leu Val
            500                 505                 510

Lys Glu Gln Trp Ile Leu Thr Ala Arg Gln Cys Phe Ser Ser Cys His
        515                 520                 525

Met Pro Leu Thr Gly Tyr Glu Val Trp Leu Gly Thr Leu Phe Gln Asn

530                 535                 540

Pro Gln His Gly Glu Pro Ser Leu Gln Arg Val Pro Val Ala Lys Met
545                 550                 555                 560

Val Cys Gly Pro Ser Gly Ser Gln Leu Val Leu Leu Lys Leu Glu Arg
                565                 570                 575

Ser Val Thr Leu Asn Gln Arg Val Ala Leu Ile Cys Leu Pro Pro Glu
            580                 585                 590

Trp Tyr Val Val Pro Pro Gly Thr Lys Cys Glu Ile Ala Gly Trp Gly
        595                 600                 605

Glu Thr Lys Gly Thr Gly Asn Asp Thr Val Leu Asn Val Ala Leu Leu
    610                 615                 620

Asn Val Ile Ser Asn Gln Glu Cys Asn Ile Lys His Arg Gly Arg Val
625                 630                 635                 640

Arg Glu Ser Glu Met Cys Thr Glu Gly Leu Leu Ala Pro Val Gly Ala
                645                 650                 655

Cys Glu Gly Asp Tyr Gly Gly Pro Leu Ala Cys Phe Thr His Asn Cys
            660                 665                 670

Trp Val Leu Glu Gly Ile Ile Ile Pro Asn Arg Val Cys Ala Arg Ser
        675                 680                 685

Arg Trp Pro Ala Val Phe Thr Arg Val Ser Val Phe Val Asp Trp Ile
    690                 695                 700

His Lys Val Met Arg Leu Gly
705                 710

<210>  9
<211>  760
<212>  PRT
<213>  Homo sapiens

<400>  9

Met Met Asp Gln Ala Arg Ser Ala Phe Ser Asn Leu Phe Gly Gly Glu
1               5                   10                  15

Pro Leu Ser Tyr Thr Arg Phe Ser Leu Ala Arg Gln Val Asp Gly Asp
            20                  25                  30

Asn Ser His Val Glu Met Lys Leu Ala Val Asp Glu Glu Glu Asn Ala

35                  40                  45

Asp Asn Asn Thr Lys Ala Asn Val Thr Lys Pro Lys Arg Cys Ser Gly
    50                  55                  60

Ser Ile Cys Tyr Gly Thr Ile Ala Val Ile Val Phe Phe Leu Ile Gly
65                  70                  75                  80

Phe Met Ile Gly Tyr Leu Gly Tyr Cys Lys Gly Val Glu Pro Lys Thr
                85                  90                  95

Glu Cys Glu Arg Leu Ala Gly Thr Glu Ser Pro Val Arg Glu Glu Pro
            100                 105                 110

Gly Glu Asp Phe Pro Ala Ala Arg Arg Leu Tyr Trp Asp Asp Leu Lys
        115                 120                 125

Arg Lys Leu Ser Glu Lys Leu Asp Ser Thr Asp Phe Thr Gly Thr Ile
    130                 135                 140

Lys Leu Leu Asn Glu Asn Ser Tyr Val Pro Arg Glu Ala Gly Ser Gln
145                 150                 155                 160

Lys Asp Glu Asn Leu Ala Leu Tyr Val Glu Asn Gln Phe Arg Glu Phe
                165                 170                 175

Lys Leu Ser Lys Val Trp Arg Asp Gln His Phe Val Lys Ile Gln Val
            180                 185                 190

Lys Asp Ser Ala Gln Asn Ser Val Ile Ile Val Asp Lys Asn Gly Arg
        195                 200                 205

Leu Val Tyr Leu Val Glu Asn Pro Gly Gly Tyr Val Ala Tyr Ser Lys
    210                 215                 220

Ala Ala Thr Val Thr Gly Lys Leu Val His Ala Asn Phe Gly Thr Lys
225                 230                 235                 240

Lys Asp Phe Glu Asp Leu Tyr Thr Pro Val Asn Gly Ser Ile Val Ile
                245                 250                 255

Val Arg Ala Gly Lys Ile Thr Phe Ala Glu Lys Val Ala Asn Ala Glu
            260                 265                 270

Ser Leu Asn Ala Ile Gly Val Leu Ile Tyr Met Asp Gln Thr Lys Phe
        275                 280                 285

Pro Ile Val Asn Ala Glu Leu Ser Phe Phe Gly His Ala His Leu Gly
    290                 295                 300

Thr Gly Asp Pro Tyr Thr Pro Gly Phe Pro Ser Phe Asn His Thr Gln
305                 310                 315                 320

Phe Pro Pro Ser Arg Ser Ser Gly Leu Pro Asn Ile Pro Val Gln Thr
                325                 330                 335

Ile Ser Arg Ala Ala Ala Glu Lys Leu Phe Gly Asn Met Glu Gly Asp
            340                 345                 350

Cys Pro Ser Asp Trp Lys Thr Asp Ser Thr Cys Arg Met Val Thr Ser
        355                 360                 365

Glu Ser Lys Asn Val Lys Leu Thr Val Ser Asn Val Leu Lys Glu Ile
    370                 375                 380

Lys Ile Leu Asn Ile Phe Gly Val Ile Lys Gly Phe Val Glu Pro Asp
385                 390                 395                 400

His Tyr Val Val Val Gly Ala Gln Arg Asp Ala Trp Gly Pro Gly Ala
                405                 410                 415

Ala Lys Ser Gly Val Gly Thr Ala Leu Leu Leu Lys Leu Ala Gln Met
            420                 425                 430

Phe Ser Asp Met Val Leu Lys Asp Gly Phe Gln Pro Ser Arg Ser Ile
        435                 440                 445

Ile Phe Ala Ser Trp Ser Ala Gly Asp Phe Gly Ser Val Gly Ala Thr
    450                 455                 460

Glu Trp Leu Glu Gly Tyr Leu Ser Ser Leu His Leu Lys Ala Phe Thr
465                 470                 475                 480

Tyr Ile Asn Leu Asp Lys Ala Val Leu Gly Thr Ser Asn Phe Lys Val
                485                 490                 495

Ser Ala Ser Pro Leu Leu Tyr Thr Leu Ile Glu Lys Thr Met Gln Asn
            500                 505                 510

Val Lys His Pro Val Thr Gly Gln Phe Leu Tyr Gln Asp Ser Asn Trp
        515                 520                 525

Ala Ser Lys Val Glu Lys Leu Thr Leu Asp Asn Ala Ala Phe Pro Phe
    530                 535                 540

Leu Ala Tyr Ser Gly Ile Pro Ala Val Ser Phe Cys Phe Cys Glu Asp
545                 550                 555                 560

Thr Asp Tyr Pro Tyr Leu Gly Thr Thr Met Asp Thr Tyr Lys Glu Leu
                565                 570                 575

Ile Glu Arg Ile Pro Glu Leu Asn Lys Val Ala Arg Ala Ala Ala Glu
            580                 585                 590

Val Ala Gly Gln Phe Val Ile Lys Leu Thr His Asp Val Glu Leu Asn
        595                 600                 605

Leu Asp Tyr Glu Arg Tyr Asn Ser Gln Leu Leu Ser Phe Val Arg Asp
    610                 615                 620

Leu Asn Gln Tyr Arg Ala Asp Ile Lys Glu Met Gly Leu Ser Leu Gln
625                 630                 635                 640

Trp Leu Tyr Ser Ala Arg Gly Asp Phe Phe Arg Ala Thr Ser Arg Leu
                645                 650                 655

Thr Thr Asp Phe Gly Asn Ala Glu Lys Thr Asp Arg Phe Val Met Lys
            660                 665                 670

Lys Leu Asn Asp Arg Val Met Arg Val Glu Tyr His Phe Leu Ser Pro
        675                 680                 685

Tyr Val Ser Pro Lys Glu Ser Pro Phe Arg His Val Phe Trp Gly Ser
    690                 695                 700

Gly Ser His Thr Leu Pro Ala Leu Leu Glu Asn Leu Lys Leu Arg Lys
705                 710                 715                 720

Gln Asn Asn Gly Ala Phe Asn Glu Thr Leu Phe Arg Asn Gln Leu Ala
                725                 730                 735

Leu Ala Thr Trp Thr Ile Gln Gly Ala Ala Asn Ala Leu Ser Gly Asp
            740                 745                 750

Val Trp Asp Ile Asp Asn Glu Phe
        755                 760